# EUROPEAN PATENT APPLICATION

(11) **EP 4 613 286 A1**
(43) Date of publication of application: **10.09.2025**
(21) Application number: 23885141.4
(22) Date of filing: 06.11.2023
(51) Int. Cl.: A61K 38/49, A61K 38/43, A61P 25/28, A61P 21/00

(54) **METHOD FOR PROMOTING PATHOLOGICAL TDP-43 PROTEIN DEGRADATION, AND DRUG**

(30) Priority: 04.11.2022 WO PCT/CN2022/129819
(71) Applicant: Talengen International Limited, Hong Kong 999077 (HK)
(72) Inventor: LI, Jinan, Beijing 100089 (CN)
(74) Representative: CMS Cameron McKenna Nabarro Olswang LLP
(86) International application number: PCT/CN2023/130019
(87) International publication number: WO 2024/094217

(57) **Abstract**

The present application relates to a method of promoting pathological TDP-43 protein degradation, which comprises administering to a subject a therapeutically effective amount of one or more compounds among: a plasminogen activation pathway component, a compound capable of directly activating plasminogen or indirectly activating plasminogen by means of activating a plasminogen activation pathway upstream component, a compound simulating the activity of plasminogen or plasmin, a compound capable of upregulating plasminogen or plasminogen activator expression, a plasminogen analogue, a plasmin analogue, a tPA or uPA analogue, and a fibrinolysis inhibitor antagonist. The present application also relates to a drug for promoting pathological TDP-43 protein degradation and a use thereof.

## Description

### TECHNICAL FIELD

The present application relates to a method for promoting the degradation of pathological TDP-43 protein and treating a pathological TDP-43 protein-related disease, comprising administering an effective amount of a plasminogen activation pathway-related compound such as plasminogen or plasmin to a subject. The present application further relates to a pharmaceutical composition for such use, wherein the pharmaceutical composition comprises a plasminogen activation pathway-related compound such as plasminogen or plasmin.

### BACKGROUND ART

TDP-43, the full name of which is Transactive response DNA-binding protein 43, is a protein widely present in cells. It can bind to DNA and RNA and play an important role in RNA transcription, alternative splicing, and mRNA stability regulation in cells. Normal TDP-43 is located in various subcellular structures, including mitochondria, mitochondrial-associated membranes, RNA granules, and stress granules, to regulate endoplasmic reticulum-mitochondrial binding, mitochondrial protein translation, and mRNA transport and translation. Therefore, the normal physiological function of TDP-43 is particularly important for cell survival.

TDP-43 can also enable protein-protein interactions to form homodimers and multimers. There is a glutamine-rich region at the C-terminus of TDP-43 that is responsible for most of the aggregation. Recent studies have found that, several factors affect the aggregation process of TDP-43, which either change the protein structure itself or change the proteins in the surrounding environment, thereby causing TDP-43 aggregation.

TDP-43 aggregates have been identified in an increasing number of neurodegenerative disorders (Lagier-Tourenne et al., Human Molecular Genetics, 2010, Vol. 19, Review Issue 1 R46-R64), including but not limited to: frontotemporal dementia (sporadic or familial, with or without motor neuron disease (MND), with progranulin (GRN) mutation, with TARDBP mutation, with valosine-containing protein (VCP) mutation, linked to chromosome 9p, corticobasal degeneration, frontotemporal degeneration with ubiquitin-positive inclusions, argyrophilic grain disease, Pick's disease, etc.), amyotrophic lateral sclerosis (sporadic ALS, with TARDBP mutation, with angiogenin (ANG) mutation), Alzheimer's disease (AD, sporadic and familial), Down syndrome, familial British dementia, polyglutamine diseases (Huntington's disease and spinocerebellar ataxia type 3 (SCA3; also known as Machado-Joseph Disease)), hippocampal sclerosis dementia, and myopathies (sporadic inclusion body myositis; inclusion body myopathy with mutations in valosin-containing protein (VCP); and Paget disease of bone and frontotemporal dementia); oculopharyngeal muscular dystrophy with rimmed vacuoles; and myofibrillar myopathy

with mutations in the myotilin (MYOT) gene or mutations in the gene encoding desmin (DES).

Aggregated TDP-43 from patient brains shows a number of abnormal modifications, including hyperphosphorylation, ubiquitination, acetylation, and C-terminal fragmentation cleaved by proteolysis (Arai et al., Biochemical and Biophysical Research Communications 351 (2006) 602-611; Neumann et al., Science 314, (2006), 130-133; Neumann et al., Acta Neuropathol. (2009) 117: 137-149; Hasegawa et al., (2008) Annals of Neurology Vol 64 No 1, 60-70; Cohen et al., Nat Commun. 6: 5845, 2015). Another characteristic feature of TDP-43 pathological conditions is the redistribution and accumulation of TDP-43 from the nucleus to the cytoplasm. The hallmark lesions of FTLD-TDP are neuronal cytoplasmic inclusions (NCI) and glial cytoplasmic inclusions (GCI), and dystrophic neurites (DN).

Frontotemporal dementia (FTD) is a clinical term that encompasses a broad spectrum of disorders based on degeneration of the frontal and temporal lobes - a pathological feature known as frontotemporal lobar degeneration (FTLD). FTD is the second leading cause of early degenerative dementia in the age group under 65 years old (Le Ber, Revue Neurologique 169 (2013) 811-819). FTD manifests as several syndromes, including bvFTD, which is characterized by personality and behavioral changes; semantic dementia (SD) and progressive nonfluent aphasia (PNFA), which are characterized by changes in language function; and corticobasal syndrome (CBS), progressive supranuclear palsy syndrome, and motor neurone disease (FTD-MND), which are characterized by motor dysfunction.

Amyotrophic lateral sclerosis (ALS) is a neurodegenerative disease characterized by the premature loss of upper and lower motor neurons. The progression of ALS is characterized by fatal paralysis and respiratory failure, with a course from diagnosis to death ranging from 1 to 5 years. In most cases of sporadic ALS, the neuropathology is characterized by abnormal cytoplasmic accumulation of TDP-43 in neurons and glial cells of the primary motor cortex, brainstem motor nuclei, spinal cord, and associated white matter tracts. ALS with dementia involves accumulation of TDP-43 in the extra-motor neocortex and hippocampus. The role of TDP-43 phosphorylation in ALS patients has been explored with the help of antibodies (Hasegawa et al., Ann Neurol 2008; 64: 60-70; Neumann et al., Acta Neuropathol (2009) 117: 137-149).

TDP-43 pathology occurs in the brains of up to 57% of patients with Alzheimer's disease (Josephs KA et al., Acta Neuropathol. 2014; 127(6): 811-824; Josephs KA et al., Acta Neuropathol. 2014; 127(3): 441-450; McAleese et al., Brain Pathol. 2017 Jul; 27(4): 472-479). TDP-43 aggregation is associated with patient age, and is associated with cognitive decline, memory loss, and mesial temporal atrophy in AD. TDP-43-positive patients were 10 times more likely to have cognitive impairment when they die compared to TDP-43-negative subjects. Pathological TDP-43 follows a general progressive deposition pattern, with TDP-43 first depositing in the amygdala (stage I), then in the hippocampus, limbic, temporal, and finally frontostriatum (stage V) (Josephs KA et al., Acta Neuropathol. 2014; 127(6):811-824; Josephs KA et al., Acta Neuropathol. 2014; 127(3):441-450).

Recent evidence supports the concept that amyloid-β, tau, α-synuclein, and TDP-43 spread proteins in neuronal tissues via a prion-like mechanism (Hasegawa et al., 2017). Although ALS onset and initial symptoms vary significantly between patients, a common feature of disease progression is the spread of pathology from the initial focal area to the majority of neurons. The continued worsening of symptoms can be explained by this progressive spread of TDP-43 pathology. TDP-43 pathology in the brains of ALS patients was shown to spread in a four-stage process, and is thought to perform transmission through synapses via corticofugal axonal projections by using anterograde axonal transport (Brettschneider et al., Ann Neurol. 2013 July; 74(1): 20-38.).

Several recent reports have involved the spread of TDP-43 at the molecular level in various in vitro models. Insoluble TDP-43 preparations from patient brains can induce intracellular aggregate formation in vitro (Nonaka et al., Cell Reports 4 (2013), 124-134; Feiler et al., 2015; Porta et al., Nat. Comm., 2018). Furthermore, it has recently been shown that patient-derived pathological TDP-43 can lead to extensive deposition of endogenous TDP-43 after inoculation into transgenic and wild-type mice (Porta et al., Nat. Comm., 2018). Furthermore, it has been shown that intracellular TDP-43 aggregates are released in conjunction with exosomes before spreading to the next cell (Nonaka et al., Cell Reports 4 (2013, 124-134)).

TDP-43 aggregation and pathological spread are the main hallmarks of ALS and FTD as currently incurable and fatal diseases. Mutations in TDP-43 have been associated with familial cases of ALS and FTD, providing a causal link between TDP-43 misfolding and disease progression. Therefore, there is a need to find methods for promoting the degradation of pathological TDP-43 protein or reducing TDP-43 aggregates in order to treat diseases associated with pathological TDP-43 protein.

### SUMMARY

The study in the present application found that plasminogen can promote the degradation of pathological TDP-43 protein in nerve and muscle tissues to some extent, and treat diseases associated with pathological TDP-43 protein aggregation, such as ALS and frontotemporal dementia.

Particularly, this application relates to the following:
1. A method for promoting degradation of pathological TDP-43 protein, comprising administering to a subject a therapeutically effective amount of one or more compounds selected from the group consisting of: a component of the plasminogen activation pathway, a compound capable of directly activating plasminogen or indirectly activating plasminogen by activating a upstream component of the plasminogen activation pathway, a compound simulating the activity of plasminogen or plasmin, a compound capable of upregulating the expression of plasminogen or plasminogen activator, a plasminogen analog, a plasmin analog, a tPA or uPA analog, and an antagonist of fibrinolysis inhibitor.
2. The method according to item 1, wherein the component of the plasminogen activation pathway is selected from the group consisting of plasminogen, recombinant human plasmin, Lys-plasminogen, Glu-plasminogen, plasmin, plasminogen and plasmin variants and analogs comprising one or more kringle domains and a protease domain of plasminogen and plasmin, mini-plasminogen, mini-plasmin, micro-plasminogen, micro-plasmin, delta-plasminogen, delta-plasmin, plasminogen activator, tPA and uPA.
3. The method according to item 1, wherein the antagonist of the fibrinolysis inhibitor is an inhibitor of PAI-1, complement C1 inhibitor, α2 antiplasmin or α2 macroglobulin, such as an antibody.
4. The method according to any one of items 1-3, wherein the compound has one or more activities selected from the group consisting of: promoting the degradation of pathological TDP-43 protein in nerve tissue, promoting the degradation of pathological TDP-43 protein in muscle tissue, and promoting the degradation of TDP-43 in cells and cell nuclei.
5. A method for treating a pathological TDP-43 protein-related disease in a subject, comprising administering to the subject a therapeutically effective amount of one or more compounds selected from the group consisting of: a component of the plasminogen activation pathway, a compound capable of directly activating plasminogen or indirectly activating plasminogen by activating a upstream component of the plasminogen activation pathway, a compound simulating the activity of plasminogen or plasmin, a compound capable of upregulating the expression of plasminogen or plasminogen activator, a plasminogen analog, a plasmin analog, a tPA or uPA analog, and an antagonist of fibrinolysis inhibitor, wherein the pathological TDP-43 protein-related disease is one or more selected from the group consisting of: amyotrophic lateral sclerosis (ALS), bulbar amyotrophic lateral sclerosis, Fus gene mutation amyotrophic lateral sclerosis, Alzheimer's disease, argyrophilic grain disease, ALS-parkinsonism dementia complex of Guam, vascular dementia, frontotemporal dementia (FTD), semantic dementia, dementia with Lewy bodies, Huntington's disease, spinocere bellarataxia, inclusion body myopathy, inclusion body myositis, and Parkinson's disease.
6. The method according to item 5, wherein the component of the plasminogen activation pathway is selected from the group consisting of: plasminogen, recombinant human plasmin, Lys-plasminogen, Glu-plasminogen, plasmin, plasminogen and plasmin variants and analogs comprising one or more kringle domains and a protease domain of plasminogen and plasmin, mini-plasminogen, mini-plasmin, micro-plasminogen, micro-plasmin, delta-plasminogen, delta-plasmin, plasminogen activator, tPA and uPA.
7. The method according to item 5, wherein the antagonist of the fibrinolysis inhibitor is an inhibitor of PAI-1, complement C1 inhibitor, α2 antiplasmin or α2 macroglobulin, such as an antibody.
8. The method according to any one of items 1-7, wherein the compound is plasminogen or plasmin.
9. The method according to any one of items 1-8, wherein the plasminogen is Glu-plasminogen, Lys-plasminogen, or a conservatively substituted variant thereof.
10. The method according to any one of items 1-9, wherein the plasminogen has at least 75%, 80%, 85%, 90%, 95%, 96%, 97%, 98% or 99% sequence identity with SEQ ID NO: 2 and has lysine binding activity and/or proteolytic activity of plasminogen.
11. The method according to any one of items 1-10, wherein the plasminogen comprises one or more domains selected from the group consisting of:
   1) a serine protease domain represented by SEQ ID NO:14;
   2) a serine protease domain having at least 80%, 90%, 95%, 96%, 97%, 98%, 99% identity with SEQ ID NO: 14 and retaining proteolytic activity;
   3) one or more Kringle domains selected from the group consisting of: Kringle 1, Kringle 2, Kringle 3, Kringle 4 and Kringle 5; and
   4) a Kringle domain having at least 80%, 90%, 95%, 96%, 97%, 98%, 99% identity with one or more of Kringle 1, Kringle 2, Kringle 3, Kringle 4, and Kringle 5 and retaining lysine binding activity.
12. The method according to any one of items 1-11, wherein the plasminogen is selected from Glu-plasminogen, Lys-plasminogen, mini-plasminogen, micro-plasminogen, delta-plasminogen, or variants thereof retaining the proteolytic activity of plasminogen.
13. The method according to any one of items 1-12, wherein the plasminogen comprises an amino acid sequence represented by SEQ ID NO: 2, 6, 8, 10 or 12, or a conservatively substituted variant of the amino acid sequence represented by SEQ ID NO: 2, 6, 8, 10 or 12.
14. The method according to any one of items 1-13, wherein the plasminogen is used in combination with one or more other therapies or drugs.
15. The method according to item 14, wherein the other therapies include cell therapy (including stem cell therapy), supportive therapy and physical therapy.
16. The method according to any one of items 1-15, wherein the plasminogen is administered by nasal inhalation, nebulized inhalation, nasal drops, eye drops, ear drops, or intravenous, intraperitoneal, subcutaneous, sublingual, intracranial, intrathecal, intraarterial or intramuscular administration. In some particular embodiments, the plasminogen pathway activator is administered in combination with one or more other drugs and/or treatments, preferably the treatments include cell therapy (e.g., stem cell therapy) and gene therapy, such as antisense RNA, small molecule splicing modifiers.

In some particular embodiments, the plasminogen pathway activator is a component of the plasminogen activation pathway, such as plasminogen. In some particular embodiments, the plasminogen comprises or has an amino acid sequence that has at least 75%, 80%, 85%, 90%, 95%, 96%, 97%, 98% or 99% sequence identity with the amino acid sequence represented by SEQ ID NO: 2, 6, 8, 10 or 12, and has plasminogen activity and/or lysine binding activity. In some embodiments, the plasminogen is a protein with an addition, deletion, and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid based on SEQ ID NO: 2, 6, 8, 10 or 12, and has plasminogen activity and/or lysine binding activity. In some particular embodiments, the plasminogen activity is the proteolytic activity of plasminogen. In some particular embodiments, the plasminogen is a protein comprising a plasminogen active fragment and having plasminogen activity and/or lysine binding activity. In particular some embodiments, the plasminogen activity is the proteolytic activity of plasminogen. In some particular embodiments, the plasminogen active fragment comprises or has a plasminogen serine protease domain or a plasminogen protease domain. In some particular embodiments, the amino acid sequence of the plasminogen active fragment is represented by SEQ ID NO:14. In some particular embodiments, the plasminogen is selected from Glu-plasminogen (human full-length plasminogen), Lys-plasminogen (human full-length plasminogen after cleavage between amino acids 76-77), mini-plasminogen (comprising Kringle 5 (K5) and a serine protease domain), micro-plasminogen (comprising a serine protease domain), delta-plasminogen (comprising Kringle 1 and a serine protease domain), or variants thereof that retain plasminogen activity. In some particular embodiments, the plasminogen is human full-length plasminogen, or a variant or fragment thereof that still retains plasminogen activity and/or lysine binding activity. In some embodiments, the plasminogen is an ortholog of human plasminogen from a primate or a rodent, or a variant or fragment thereof that still retaining plasminogen activity and/or lysine binding activity. In some embodiments, the plasminogen comprises the amino acid sequence represented by SEQ ID NO: 2, 6, 8, 10 or 12. In some embodiments, the plasminogen is human natural plasminogen.

In some particular embodiments, the plasminogen pathway activator is administered systemically or locally, e.g., administered intravenously, intramuscularly, or by nasal inhalation, nebulized inhalation, by nasal drops. In some embodiments, the subject is a human. In some embodiments, the subject lacks or is deficient in plasminogen. In some embodiments, the lack or deficiency is congenital, secondary and/or local. In some embodiments, the plasminogen is administrated daily, or every second day or every third day consecutively at a dose of 0.0001-2000 mg/kg, 0.001-800 mg/kg, 0.01-600 mg/kg, 0.1-400 mg/kg, 1-200 mg/kg, 1-100 mg/kg, 10-100mg/kg (calculating by per kilogram of body weight); or 0.0001-2000 mg/cm², 0.001-800 mg/cm², 0.01-600 mg/cm², 0.1-400 mg/cm², 1-200 mg/cm², 1-100 mg/cm², 10-100 mg/cm² (calculating by per square centimeter of body surface area).

In one aspect, the present application further relates to a pharmaceutical composition, medicament, preparation, kit, and product used in the above method, which comprises the above plasminogen pathway activator, e.g., the above plasminogen.

In some embodiments, the pharmaceutical composition, medicament, preparation comprises a pharmaceutically acceptable carrier and a plasminogen pathway activator, for example, a component of the plasminogen activation pathway, e.g., plasminogen. In some embodiments, the kit or product comprises one or more containers containing the pharmaceutical composition, medicament or preparation therein. In some embodiments, the kit or product further comprises a label or instructions for use indicating that the plasminogen pathway activator, for example, a component of the plasminogen activation pathway, e.g., plasminogen is used in the above method. In some embodiments, the kit or product further comprises one or more additional containers containing one or more additional drugs.

In one aspect, the present application further relates to a plasminogen pathway activator, such as plasminogen as described above, for the use as described above.

In one aspect, the present application further relates to use of the therapeutically effective amount of the above plasminogen pathway activator in the preparation of a pharmaceutical composition, medicament, preparation, kit, and product for the above methods.

In some embodiments, the plasminogen pathway activator is one or more selected from the group consisting of: a component of plasminogen activation pathway, a compound directly activating plasminogen or indirectly activating plasminogen by activating an upstream component of plasminogen activation pathway, a compound simulating the activity of plasminogen or plasmin, a compound capable of upregulating the expression of plasminogen or plasminogen activator, a plasminogen analog, a plasmin analog, a tPA or uPA analog, and an antagonist of fibrinolysis inhibitor.

In some particular embodiments, the component of plasminogen activation pathway is selected from the group consisting of: plasminogen, recombinant human plasmin, Lys-plasminogen, Glu-plasminogen, plasmin, plasminogen and plasmin variants and analogs comprising one or more kringle domains and/or a protease domain of plasminogen and plasmin, mini-plasminogen, mini-plasmin, micro-plasminogen, micro-plasmin, delta-plasminogen, delta-plasmin, plasminogen activator, tPA and uPA. In some particular embodiments, the antagonist of fibrinolysis inhibitor is an antagonist of PAI-1, complement C1 inhibitor, α2 antiplasmin or α2 macroglobulin, e.g., an antibody of PAI-1, complement C1 inhibitor, α2 antiplasmin or α2 macroglobulin.

In some particular embodiments, the plasminogen pathway activator is administered in combination with one or more other drugs and/or therapies, preferably, the therapies include cell therapy (e.g., stem cell therapy) and gene therapy, such as antisense RNA, small molecule splicing modifiers.

In some particular embodiments, the plasminogen pathway activator is a component of the plasminogen activation pathway, e.g., plasminogen. In some particular embodiments, the plasminogen comprises or has an amino acid sequence having at least 75%, 80%, 85%, 90%, 95%, 96%, 97%, 98% or 99% sequence identity with the amino acid sequence represented by SEQ ID NO: 2, 6, 8, 10 or 12, and has the plasminogen activity and/or lysine binding activity. In some embodiments, the plasminogen is a protein with an addition, deletion, and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid based on SEQ ID NO: 2, 6, 8, 10 or 12, and having plasminogen activity and/or lysine binding activity. In some particular embodiments, the plasminogen activity is the proteolytic activity of plasminogen. In some particular embodiments, the plasminogen is a protein comprising an active fragment of plasminogen and having plasminogen activity and/or lysine binding activity. In some particular embodiments, the plasminogen activity is the proteolytic activity of plasminogen. In some particular embodiments, the active fragment of plasminogen comprises or has a serine protease domain of plasminogen or a plasminogen protease domain. In some particular embodiments, the amino acid sequence of the active fragment of plasminogen is represented by SEQ ID NO: 14. In some particular embodiments, the plasminogen is selected from the group consisting of: Glu-plasminogen (human full-length plasminogen), Lys-plasminogen (a human full-length plasminogen after cleavage between amino acid positions 76-77), mini-plasminogen (comprising Kringle 5 (K5) and serine protease domain), micro-plasminogen (comprising serine protease domain), delta-plasminogen (comprising Kringle 1 and serine protease domain), or variants thereof retaining plasminogen activity. In some particular embodiments, the plasminogen is human full-length plasminogen, or a variant or fragment thereof that still retains plasminogen activity and/or lysine binding activity. In some embodiments, the plasminogen is a human plasminogen ortholog from a primate or a rodent, or a variant or fragment thereof still retaining plasminogen activity and/or lysine binding activity. In some embodiments, the plasminogen comprises the amino acid sequence represented by SEQ ID NO: 2, 6, 8, 10 or 12. In some embodiments, the plasminogen is human natural plasminogen.

In some embodiments, the plasminogen pathway activator, for example, a component of the plasminogen activation pathway, e.g., plasminogen, is administered in combination with one or more other drugs and/or therapies. In some embodiments, the plasminogen pathway activator, for example, a component of the plasminogen activation pathway, e.g. plasminogen is administered intravenously, intramuscularly, intrathecally, by nasal inhalation, nebulized inhalation, by nasal drop or eye drop.

In some embodiments, the pharmaceutical composition, medicament, preparation comprises a pharmaceutically acceptable carrier and a plasminogen pathway activator, for example, a component of the plasminogen activation pathway, e.g., plasminogen. In some embodiments, the kit or product comprises one or more containers containing the pharmaceutical composition, medicament or preparation therein. In some embodiments, the kit or product further comprises a label or instructions for use indicating that the plasminogen pathway activator, for example, a component of the plasminogen activation pathway, e.g., plasminogen is used in the above method.

In some embodiments, the kit or product further comprises one or more additional containers containing one or more additional drugs.

The present application explicitly encompasses all the combinations of the technical features belonging to the embodiments of the present application, and these combined technical solutions have been explicitly disclosed in this application, just as the above technical solutions have been separately and explicitly disclosed. In addition, the present application further explicitly encompasses the combinations of each embodiment and its elements, and the combined technical solutions are explicitly disclosed herein.

### BRIEF DESCRIPTION OF THE DRAWINGS

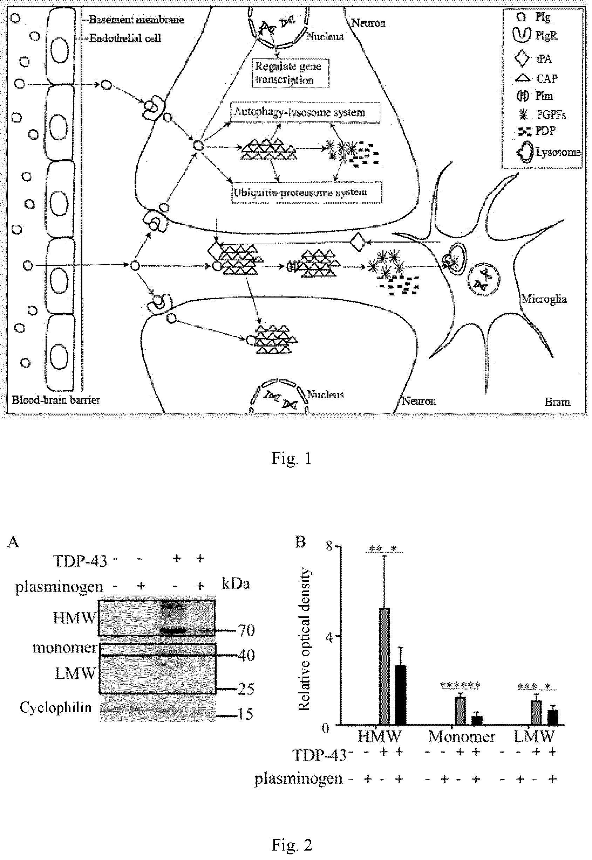

Fig. 1 is a schematic diagram of the mechanism by which plasminogen promotes the degradation of pathological proteins in the central nervous system. Blood-brain barrier, basement membrane, endothelial cells, plasminogen (Plg), plasminogen receptor (PlgR), tissue-type plasminogen activator (tPA), conformationally abnormal proteins (CAP), plasmin (Plm), plasmin generated protein fragments (PGPFs), plasmin degradation products (PDP), lysosome, ubiquitin (UBI), ubiquitin-activating enzyme (E1), ubiquitin-conjugating enzyme (E2), ubiquitin ligase (E3), proteasome, microglia, nucleus. Existing results show that, plasminogen can promote the degradation of pathological proteins in the central nervous system such as TDP-43 and superoxide dismutase-1 (SOD1), and can improve the clinical symptoms of various neurodegenerative diseases including amyotrophic lateral sclerosis. Based on the existing data, it is speculated that the mechanism by which plasminogen promotes the degradation of pathological proteins in the central nervous system is as follows: (1) Plasminogen crosses the blood-brain barrier and enters the central nervous system, where it is enriched and activated to form plasmin. Plasmin directly degrades pathological proteins in the central nervous system that are abnormally deposited in the extracellular matrix, such as amyloid Aβ. The protein fragments formed by degradation are further phagocytosed by microglia and then degraded by lysosomes. (2) Plasminogen enters the cell or the nucleus through endocytosis and is activated to form plasmin, which promotes the degradation of pathological proteins in the central nervous system, such as TDP-43, SOD1, TAU, and α-synuclein. (3) Plasminogen enters the cell and regulates function of the intracellular protein degradation system -- the ubiquitin proteasome system (UPS), and the pathological proteins in the central nervous system are degraded through UPS system. (4) Plasminogen enters the cell to regulate the function of the intracellular protein degradation system -- the autophagy-lysosome system, and the pathological proteins in the central nervous system are degraded through the autophagy-lysosome system. (5) In addition, some studies have reported that pathological proteins in the central nervous system including TDP-43 and SOD1 have similar infectivity to prions, and plasminogen may have the ability to prevent pathological proteins in the central nervous system from spreading between cells.
Figs. 2A-B show the effect of plasminogen on TDP-43 protein in normal mouse brain homogenate. Fig. 2A is a Western blot image, and Fig. 2B is the result of quantitative analysis of the optical density of TDP-43 protein bands. The results show that, the molecular weight of the recombinant TDP-43 protein monomer is about 43 kDa, the molecular weight of the high molecular weight TDP-43 protein (HMW) is >55 kDa, and the molecular weight of the low molecular weight TDP-43 fragment (LMW) is <40 kDa. In addition, in the normal mouse brain homogenate, the amounts of TDP-43 monomer, HMW and LMW in the plasminogen group are significantly lower than those in the vehicle control group, and the difference is extremely significant (*** represents P < 0.001, * represents P < 0.05). This suggests that plasminogen can promote the cleavage of TDP-43 in normal mouse brain homogenate.

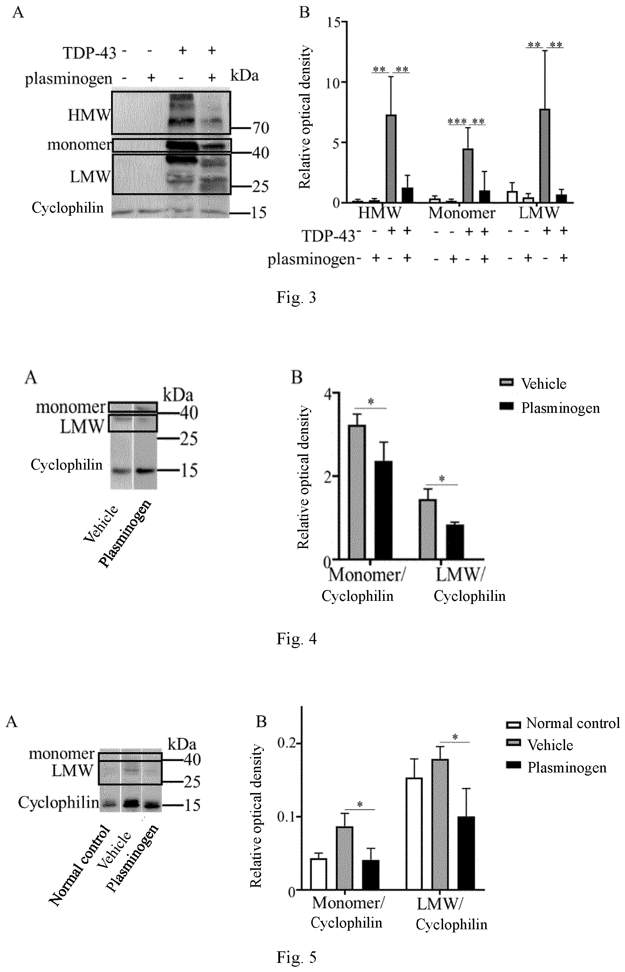

Figs. 3A-B show the effect of plasminogen on TDP-43 protein in the brain homogenate of ALS model mice. Fig. 3A is a Western blot image, and Fig. 3B is the result of quantitative analysis of the optical density of TDP-43 protein bands. The results show that, the molecular weight of the recombinant TDP-43 protein monomer is about 43 kDa, the molecular weight of the high molecular weight TDP-43 protein (HMW) is >55 kDa, and the molecular weight of the low molecular weight TDP-43 fragment (LMW) is <40 kDa. In addition, in the brain homogenate of ALS model mice, the amounts of TDP-43 monomer, HMW and LMW in the plasminogen group are significantly lower than those in the vehicle control group, and the difference is extremely significant (*** represents P < 0.001, ** represents P < 0.01). This suggests that plasminogen can promote the cleavage of TDP-43 in the brain homogenate of ALS model mice.
Figs. 4A-B show that plasminogen promotes TDP-43 protein degradation in the spinal cord tissue of ALS model mice. Fig. 4A is a Western blot image, and Fig. 4B is the result of quantitative analysis of the optical density of TDP-43 protein bands. The results show that, the amounts of TDP-43 monomer and low molecular weight TDP-43 in the spinal cord tissue of mice in the plasminogen group are significantly lower than those in the vehicle group, and the statistical difference is significant (* represents P<0.05). This indicates that plasminogen can promote the degradation of TDP-43 in the spinal cord tissue of ALS model mice.
Figs. 5A-B show that plasminogen promotes the degradation of TDP-43 protein in the brain tissue of ALS model mice injected with pathological TDP-43 protein in the brain. Fig. 5A is a Western blot image, and Fig. 5B is the result of quantitative analysis of the optical density of TDP-43 protein bands. The results show that, the amounts of TDP-43 monomer and low molecular weight TDP-43 in the brain tissue of mice in the plasminogen group are significantly lower than those in the vehicle group, and the statistical difference is significant (* represents P<0.05). This indicates that plasminogen can promote the degradation of TDP-43 in the brain tissue of ALS model mice.

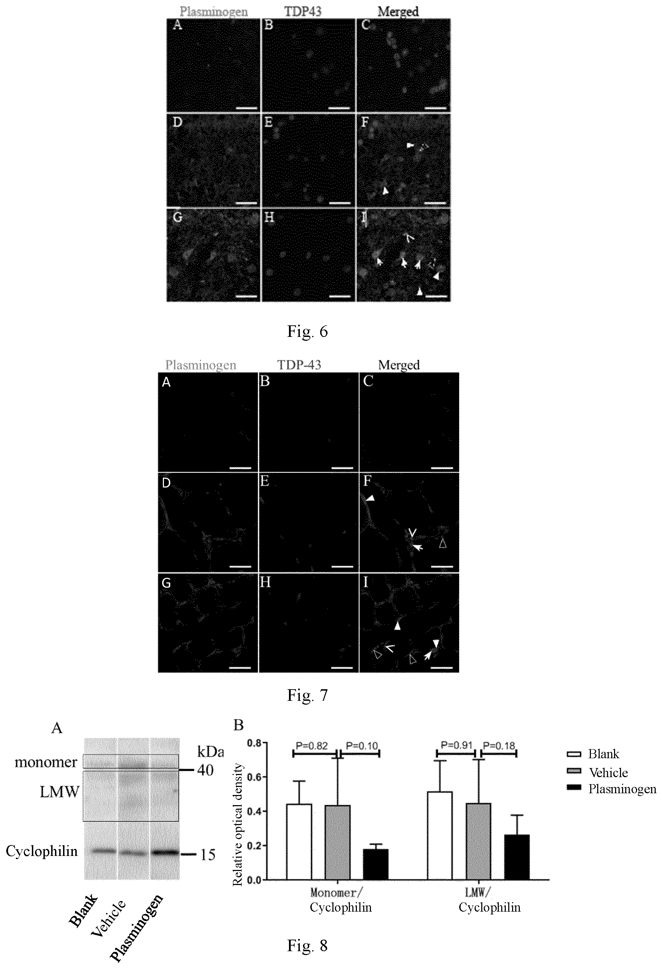

Figs. 6A-I are representative images of immunofluorescence co-localization staining of plasminogen and TDP-43 in the spinal cord tissue of mice after administration of plasminogen to ALS model mice. Figs. 6A-C: normal control group, Figs. 6D-F: vehicle group, Figs. 6G-I: plasminogen group. The results show that, the positive staining of plasminogen (green fluorescence) in the spinal cord tissue of the plasminogen group is significantly more than that of the vehicle group, indicating that the administered plasminogen can enter the spinal cord tissue and be enriched in the spinal cord tissue. In addition, plasminogen is present in the cytoplasm (as indicated by △) and in the nucleus (as indicated by 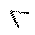 ). Plasminogen co-localizes with TDP-43 (red fluorescence) in the cytoplasm (as indicated by ▲) and in the nucleus (as indicated by 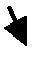 ). The level of TDP-43 in the spinal cord tissue of the plasminogen group is lower than that of the vehicle group, and the co-localization of plasminogen and TDP-43 in the plasminogen group is more than that in the vehicle group. This indicates that in ALS model mice, plasminogen can enter the spinal cord tissue, enter the cells, co-localize with TDP-43, and degrade TDP-43.
Figs. 7A-I are representative images of immunofluorescence co-localization staining of plasminogen and TDP-43 in the muscle tissue of mice after administration of plasminogen to ALS model mice. Figs. 7A-C: normal control group, Figs. 7D-F: vehicle group, Figs. 7G-I: plasminogen group. The results show that, the positive staining of plasminogen (green fluorescence) in muscle tissue in the plasminogen group is significantly more than that in the vehicle group, indicating that plasminogen can be enriched in muscle tissue. In addition, plasminogen is present in the cytoplasm (as indicated by △) and the nucleus (as indicated by 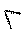 ). Plasminogen co-localizes with TDP-43 (red fluorescence) in the cytoplasm (as indicated by **▲**) and in the nucleus (as indicated by 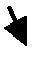 ). This indicates that in ALS model mice, plasminogen can be enriched in muscle tissue, enter cells, and co-localize with TDP-43.
Figs. 8A-B show the WB detection results of TDP-43 level in brain homogenates of mice with dementia induced by okadaic acid after administration of plasminogen. Fig. 8A is a Western blot image, and Fig. 8B is the result of quantitative analysis of the optical density of TDP-43 protein bands. The results show that, the levels of TDP-43 monomer and low molecular weight TDP-43 in the brain tissue of mice in the plasminogen group are significantly lower than those in the vehicle group. This indicates that plasminogen can promote the degradation of TDP-43 in the brain tissue of okadaic acid-induced dementia model mice.

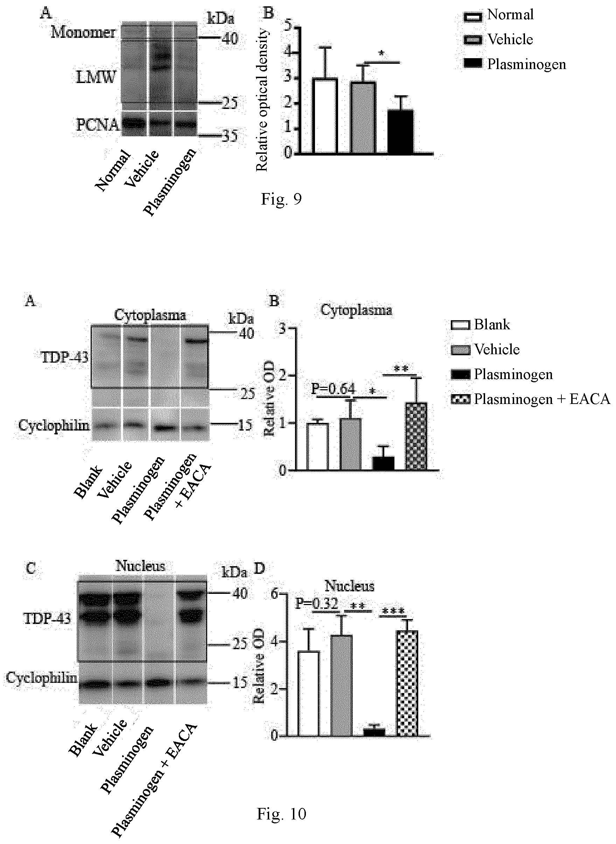

Figs. 9A-B show the WB detection results of TDP-43 level in the nuclei of renal cells in ALS model mice after administration of plasminogen. Fig. 9A is a Western blot image, and Fig. 9B is the result of quantitative analysis of the optical density of TDP-43 protein bands. The results show that, the level of TDP-43 in the renal cell nuclei of the plasminogen group is significantly lower than that of the vehicle group (* represents P<0.05). This suggests that plasminogen can promote the degradation of TDP-43 in the nucleus of renal cells.
Figs. 10A-D show the WB detection results of TDP-43 levels in the cytoplasm and nucleus of NSC34 cells treated with okadaic acid after administration of plasminogen. Fig. 10A is the Western blot image of the cytoplasm, Fig. 10B is the result of the quantitative analysis of the optical density of the cytoplasmic TDP-43 protein band, Fig. 10C is the Western blot image of the nucleus, and Fig. 10D is the result of the quantitative analysis of the optical density of the nuclear TDP-43 protein band. The results show that, the levels of TDP-43 in the cytoplasm and nucleus of the plasminogen group are almost significantly or significantly lower than those in the nucleus of the vehicle group, and the addition of EACA can completely inhibit the effect of plasminogen on TDP-43 (** represents P<0.01, *** represents P<0.001). These results suggest that plasminogen can degrade TDP-43 in the cytoplasm and nucleus, and this effect of plasminogen is closely related to the lysine binding site in its structure.

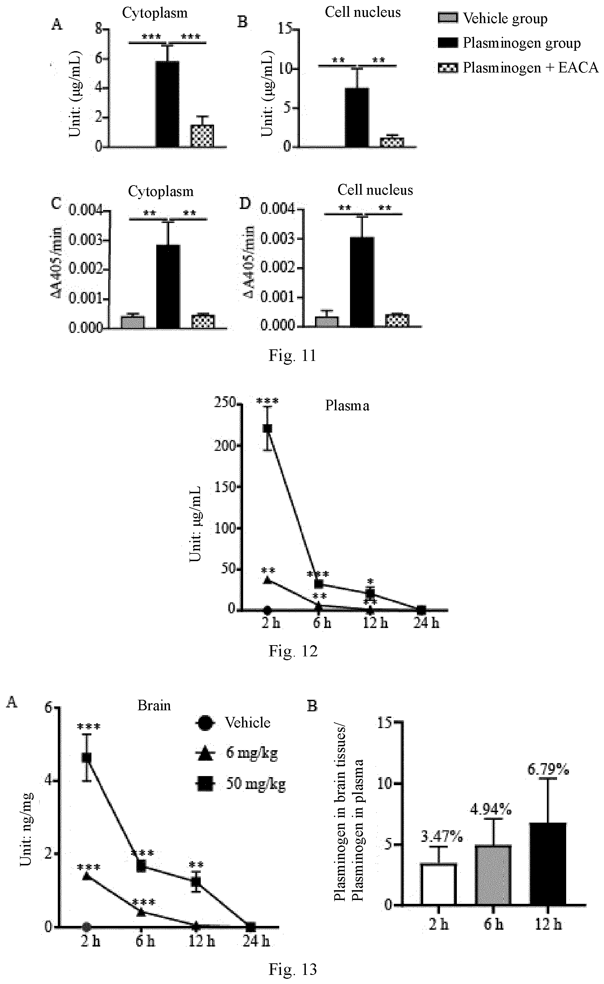

Figs. 11A-D show the detection results of plasminogen and plasmin activity levels in the cytoplasm and nucleus of NSC34 cells treated with okadaic acid after administration of plasminogen. Fig. 11A is the result of ELISA for the level of cytoplasmic plasminogen, Fig. 11B is the result of ELISA for the plasminogen level in the nucleus, Fig. 11C is the result of enzyme substrate kinetics for the level of cytoplasmic plasmin activity, and Fig. 11D is the result of enzyme substrate kinetics for the level of nuclear plasmin activity. The results show that, the levels of human plasminogen and plasmin activity in the cytoplasm and nucleus of the plasminogen group are significantly higher than those in the vehicle group, and the statistical differences are significant; the addition of EACA can completely inhibit these effects of plasminogen (** represents P<0.01, *** represents P<0.001). This suggests that plasminogen can enter cells and even the cell nucleus, increasing plasmin activity, and that the entry of plasminogen into cells and cell nuclei is closely related to its lysine binding activity.
Fig. 12 shows ELISA results of plasma plasminogen levels at different time points after tail vein injection of plasminogen into SOD1-G93A mice. The detection results of plasma ELISA level in SOD1-G93A mice show that, the plasma plasminogen levels of SOD1-G93A mice increase significantly after tail vein injection of 50 mg/kg and 6 mg/kg plasminogen, and the plasminogen level in the 50 mg/kg group is significantly higher than that in the 6 mg/kg group. Plasminogen level gradually decreases 2 hours after administration, and the plasminogen is almost completely metabolized within 12-24 hours. * represents P<0.05, ** represents P<0.01, and *** represents P<0.001. The results show that: (1) the plasminogen level in plasma has a dose-dependent effect, and the higher the concentration of plasminogen administered, the more it aggregates; (2) the plasminogen level in plasma has a time-dependent effect, first increasing and then gradually decreasing from 2 to 12 hours.
Figs. 13A-B shows the ELISA results of brain tissue plasminogen levels at different time points after intravenous injection of plasminogen into SOD1-G93A mice, as well as the ratio of plasminogen in brain tissue to plasminogen in plasma. Fig. 13A is the detection result of ELISA on the plasminogen level in brain tissue, and Fig. 13B is the ratio of plasminogen in brain tissue to plasminogen in plasma. The ELISA results of plasminogen level in the brain of SOD1-G93A mice show that the plasminogen levels in the brain tissue of SOD1-G93A mice increase significantly after tail vein injection of 50 mg/kg and 6 mg/kg plasminogen, and the plasminogen level in the 50 mg/kg group is significantly higher than that in the 6 mg/kg group. Plasminogen level gradually decreases 2 hours after administration, and the plasminogen is almost completely metabolized within 12-24 hours. The ratios of brain tissue plasminogen levels to blood plasminogen levels are 3.47%, 4.94% and 6.79% at 2, 6 and 12 hours after administration of plasminogen, respectively. The results show that: (1) under physiological and pathological conditions, administration of plasminogen can promote plasminogen to cross the blood-brain barrier and accumulate in brain tissue; (2) intravenous injection of plasminogen into mice significantly increases the plasminogen level in brain tissue; (3) the accumulation of plasminogen in brain tissue has a time-dependent effect, first increasing, then gradually decreasing from 2 to 12 hours, and plasminogen is almost completely metabolized within 12-24 hours; (4) the accumulation of plasminogen in brain tissue has a dose-dependent effect, and the higher the dose administered, the higher the plasminogen level in brain tissue. ** represents P < 0.01, and *** represents P < 0.001.

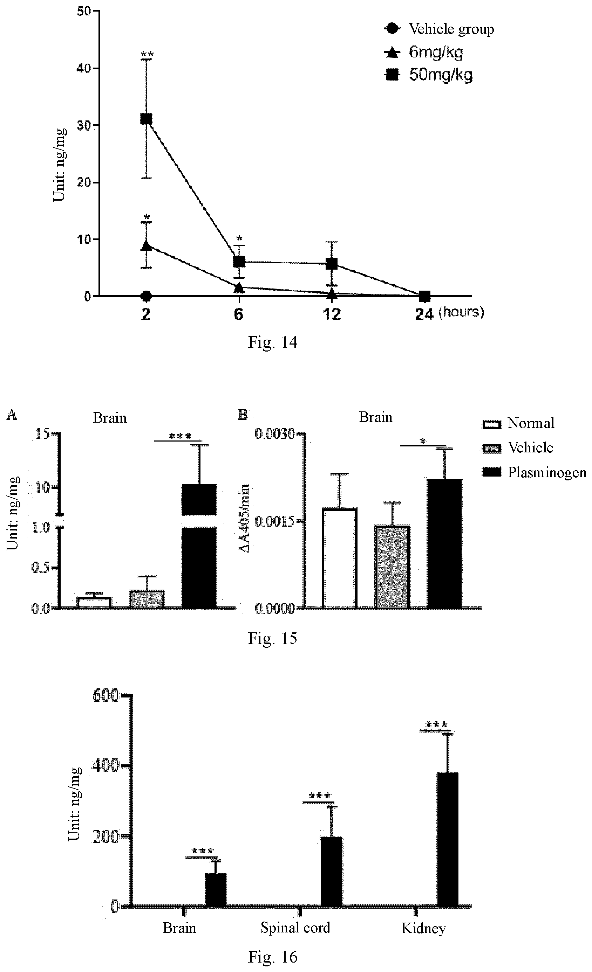

Fig. 14 shows ELISA results of spinal cord tissue plasminogen levels at different time points after intravenous injection of plasminogen into SOD1-G93A mice. The detection results of ELISA level in the spinal cord of SOD1-G93A mice show that the plasminogen levels in the spinal cord of SOD1-G93A mice increase significantly after tail vein injection of 50 mg/kg and 6 mg/kg plasminogen, and the plasminogen level in the 50 mg/kg group is significantly higher than that in the 6 mg/kg group. Plasminogen level gradually decreases 2 hours after administration, and the plasminogen is almost completely metabolized within 12-24 hours. * represents P<0.05, ** represents P<0.01. The results show that: (1) plasminogen administered at physiological dose level can cross the blood-brain barrier of SOD1-G93A mice and accumulate in the spinal cord tissue; (2) the accumulation of plasminogen in the spinal cord is dose-dependent, and the higher the dose of plasminogen administered, the greater the accumulation; and (3) the accumulation of plasminogen in the spinal cord is time-dependent, increasing first, then gradually decreasing from 2 to 12 hours, and being essentially completely metabolized within 12-24 hours.
Figs. 15A-B show the detection results of plasminogen level and plasmin activity in brain tissue homogenate after a single intravenous injection of plasminogen into SOD1-G93A mice. Fig. 15A is the detection result of ELISA for plasminogen level, and Fig. 15B is the detection result of enzyme substrate kinetic method for plasmin activity level. The results show that, the plasminogen level and plasmin activity level in the brain tissue homogenate of mice in the plasminogen group is significantly higher than that in the vehicle group, and the statistical difference is significant (* represents P<0.05, *** represents P<0.001). These results suggest that intravenous administration of plasminogen can promote increase of plasminogen level and plasmin activity in brain tissue.
Fig. 16 shows the detection result of the nuclear plasminogen level in brain tissue, spinal cord tissue and kidney tissue after continuous intravenous injection of plasminogen into SOD1-G93A mice for 7 days. The results show that, 7 days after administration of plasminogen, the levels of human plasminogen in the nuclei of brain tissue, spinal cord tissue and kidney tissue of SOD1-G93A mice in the plasminogen group are significantly higher than those in the vehicle group, and the statistical difference is extremely significant (*** represents P<0.001). These results suggest that intravenous administration of plasminogen can promote increase of the human plasminogen level in the nuclei of brain, spinal cord and kidney tissues.

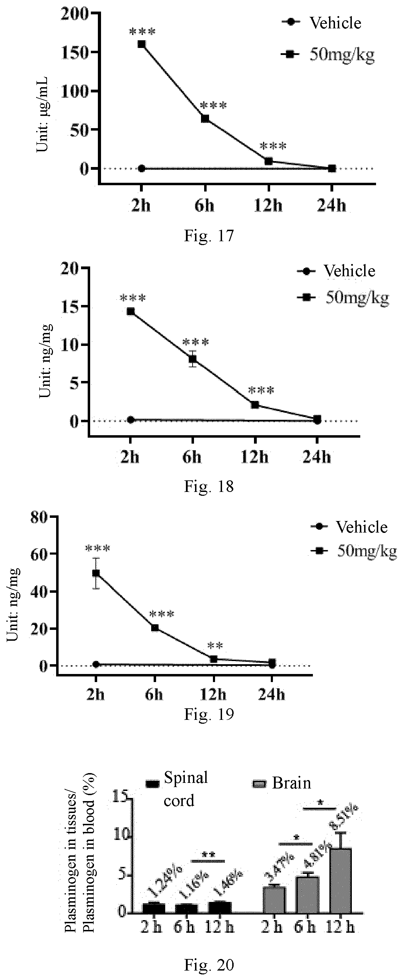

Fig. 17 shows ELISA results of blood plasminogen levels at different time points after tail vein injection of plasminogen into Parkinson's model mice. The results show that, the plasminogen level in the blood of mice in the plasminogen group is significantly higher than that in the vehicle group. The plasminogen level gradually decreases 2 hours after administration, and is almost completely metabolized within 12-24 hours. *** represents P<0.001.
Fig. 18 shows ELISA results of blood plasminogen levels at different time points after tail vein injection of plasminogen into Parkinson's model mice. The results show that, the plasminogen level in the brain tissue of mice in the plasminogen group is significantly higher than that in the vehicle group. The plasminogen level gradually decreases 2 hours after administration, and is almost completely metabolized within 12-24 hours. The results show that, plasminogen injected by tail vein can cross the blood-brain barrier and promote increase of plasminogen level in the brain tissue of Parkinson's model mice. *** represents P<0.001.
Fig. 19 shows ELISA results of plasminogen levels in the spinal cord tissue at different time points after intravenous injection of plasminogen into Parkinson's model mice. The results show that, the plasminogen level in the spinal cord tissue of mice in the plasminogen group is significantly higher than that in the vehicle group. The plasminogen level gradually decreases 2 hours after administration, and is almost completely metabolized within 12-24 hours. The results show that, plasminogen injected by tail vein can cross the blood-brain barrier and promote increase of plasminogen level in the spinal cord tissue of Parkinson's model mice. ** represents P < 0.01, and *** represents P < 0.001.
Fig. 20 shows the ratios of the plasminogen level in the spinal cord or brain tissue to the plasminogen level in the blood at different time points after tail vein injection of plasminogen into Parkinson's model mice. The results show that, the ratios of spinal cord tissue plasminogen levels to blood plasminogen levels are 1.24%, 1.16% and 1.46% at 2, 6 and 12 hours after administration of plasminogen, respectively; the ratios of brain tissue plasminogen levels to blood plasminogen levels are 3.47%, 4.18% and 8.51% at 2, 6 and 12 hours after administration of plasminogen, respectively. * represents P<0.05, ** represents P<0.01. The results show that, plasminogen injected by tail vein can cross the blood-brain barrier and promote increase of plasminogen levels in the brain and spinal cord tissues of Parkinson's model mice.

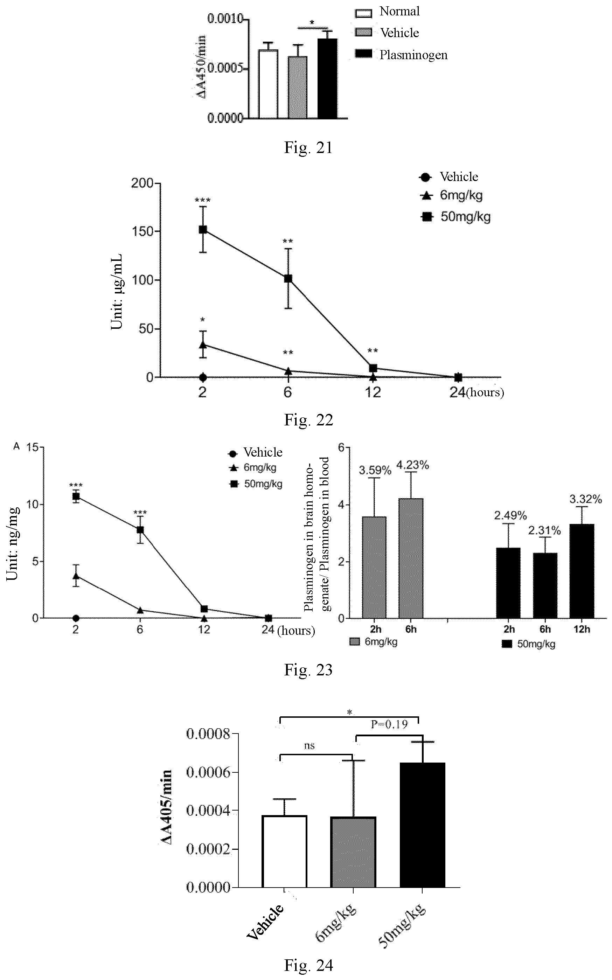

Fig. 21 shows the detection results of plasmin activity levels in brain tissue after tail vein injection of plasminogen into Parkinson's model mice. The results show that, the level of plasmin activity in the brain tissue of mice in the plasminogen group is significantly higher than that in the vehicle group, and the statistical difference is significant. *P<0.05. The results show that, plasminogen injected by tail vein can cross the blood-brain barrier and promote increase of plasmin activity level in the brain tissue of Parkinson's model mice.
Fig. 22 shows ELISA results of plasma plasminogen levels at different time points after tail vein injection of plasminogen into FAD mice. The ELISA results of plasma plasminogen level in FAD mice show that the plasma plasminogen levels of FAD mice increase significantly after tail vein injection of 50 mg/kg and 6 mg/kg plasminogen, and the plasminogen level in the 50 mg/kg group is significantly higher than that in the 6 mg/kg group. Plasminogen level gradually decreases 2 hours after administration, and the plasminogen is almost completely metabolized within 12-24 hours. The results show that: (1) the plasminogen level in plasma has a dose-dependent effect, and the higher the concentration of plasminogen administered, the more plasminogen aggregates; (2) the plasminogen level in plasma has a time-dependent effect, first increasing and then gradually decreasing from 2 to 12 hours.
Figs. 23A-B show ELISA results of brain tissue plasminogen levels at different time points after tail vein injection of plasminogen into FAD mice (Fig. 23A), and ratios of brain tissue plasminogen level to blood plasminogen level at different time points (Fig. 23B). The ELISA results of plasminogen level in the brain of FAD mice show that the plasminogen levels in the brain tissue of FAD mice increase significantly after tail vein injection of 50 mg/kg and 6 mg/kg plasminogen, and the plasminogen level in the 50 mg/kg group is significantly higher than that in the 6 mg/kg group. Plasminogen level gradually decreases 2 hours after administration, and the plasminogen is almost completely metabolized within 12-24 hours. The ratios of plasminogen level in brain tissue to plasminogen level in blood of mice in the 6 mg/kg plasminogen group are 3.59% and 4.23% at 2 and 6 hours after plasminogen injection, respectively; the ratios of plasminogen level in brain tissue to plasminogen level in blood of mice in the 50 mg/kg plasminogen group are 2.49%, 2.31% and 3.32% at 2, 6 and 12 hours after plasminogen injection, respectively. The results show that: (1) under physiological and pathological conditions, administration of plasminogen can promote plasminogen to cross the blood-brain barrier and accumulate in brain tissue; (2) intravenous injection of plasminogen into mice significantly increases the plasminogen level in brain tissue; (3) the accumulation of plasminogen in brain tissue has a time-dependent effect, first increasing, then gradually decreasing from 2 to 12 hours, and is almost completely metabolized within 12-24 hours; (4) the accumulation of plasminogen in brain tissue has a dose-dependent effect, and the higher the dose administered, the higher the plasminogen level in brain tissue.
Fig. 24 shows the detection result of the plasmin activity in brain homogenate by enzyme substrate kinetic method 2 hours after tail vein injection of plasminogen into FAD mice. The results show that, the plasminogen activity levels in the brain tissue of FAD mice increases significantly after tail vein injection of 50 mg/kg and 6 mg/kg plasminogen, and the plasminogen level in the 50 mg/kg group is significantly higher than that in the 6 mg/kg group. The results show that, the injection of plasminogen into mice significantly increases the level of plasmin in brain tissue. In addition, the plasmin activity in brain tissue has a dose-dependent effect, the higher the dose, the higher the plasminogen level in brain tissue.

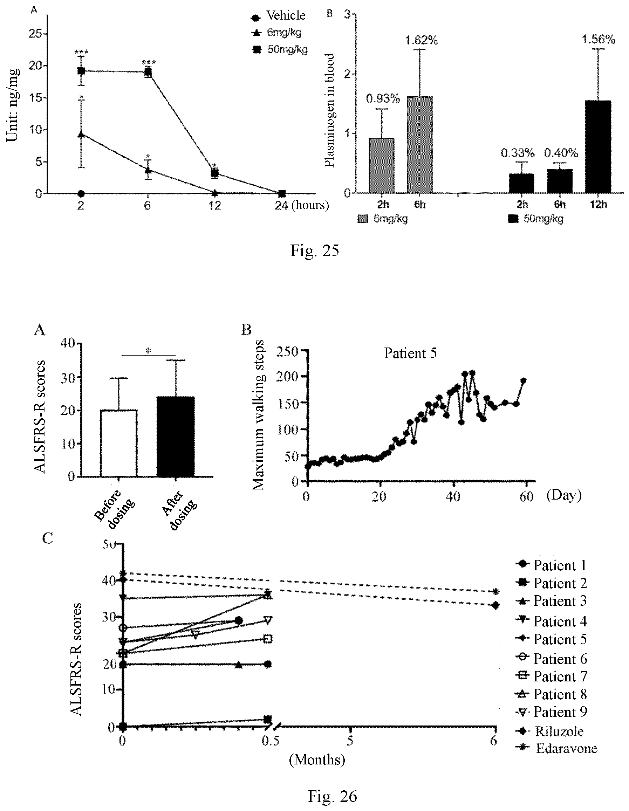

Figs. 25A-B show ELISA results of spinal cord tissue plasminogen levels at different time points after tail vein injection of plasminogen into FAD mice (Fig. 25A), and ratios of spinal cord tissue plasminogen levels to blood plasminogen levels at different time points (Fig. 25B). The ELISA results of plasminogen level in the spinal cord in FAD mice show that the plasminogen levels in the spinal cord tissue of FAD mice increases significantly after tail vein injection of 50 mg/kg and 6 mg/kg plasminogen, and the plasminogen level in the 50 mg/kg group is significantly higher than that in the 6 mg/kg group. Plasminogen level gradually decreases 2 hours after administration, and the plasminogen is almost completely metabolized within 12-24 hours. The ratios of plasminogen in the spinal cord tissue to that in the blood of the mice in the 6 mg/kg plasminogen group are 0.93% and 1.62% at 2 and 6 hours after plasminogen injection, respectively; the ratios of plasminogen in the spinal cord tissue to that in the blood of the mice in the 50 mg/kg plasminogen group are 0.33%, 0.40% and 1.56% at 2, 6 and 12 hours after plasminogen injection, respectively. The results show that: (1) under physiological and pathological conditions, administration of plasminogen can promote plasminogen to cross the blood-brain barrier and accumulate in the spinal cord tissue; (2) intravenous injection of plasminogen into mice significantly increases the plasminogen level in the spinal cord tissue; (3) the accumulation of plasminogen in the spinal cord tissue has a time-dependent effect, first increasing, then gradually decreasing from 2 to 12 hours, and is almost completely metabolized within 12-24 hours; (4) the accumulation of plasminogen in the spinal cord tissue has a dose-dependent effect, and the higher the dose administered, the higher the plasminogen level in the spinal cord tissue.
Figs. 26A-C show the changes in clinical phenotype of ALS patients before and after administration of plasminogen. Fig. 26A: ALSFRS-R scores of 9 ALS patients before and after administration of plasminogen, Fig. 26B: the maximum number of steps walked by patient 5 during the second course of medication, Fig. 26C: ALSFRS-R scores of 9 ALS patients taking plasminogen for 0.5 months (represented by the solid line), and ALSFRS-R scores of ALS patients taking Riluzole or Edaravone for 6 months (represented by the dotted line).

### DETAILED DESCRIPTION

Fibrinolytic system is a system consisting of a series of chemical substances involved in the process of fibrinolysis, mainly including plasminogen, plasmin, plasminogen activator, and fibrinolysis inhibitor. Plasminogen activators include tissue-type plasminogen activator (t-PA) and urokinase-type plasminogen activator (u-PA). t-PA is a serine protease that is synthesized by vascular endothelial cells. t-PA activates plasminogen, which is mainly carried out on fibrin; urokinase-type plasminogen activator (u-PA) is produced by renal tubular epithelial cells and

vascular endothelial cells, and may directly activate plasminogen without the need for fibrin as a cofactor. Plasminogen (PLG) is synthesized by the liver. When blood coagulates, a large amount of PLG is adsorbed on the fibrin network, and under the action of t-PA or u-PA it is activated into plasmin to promote fibrinolysis. Plasmin (PL) is a serine protease whose functions are as follows: degrading fibrin and fibrinogen; hydrolyzing various coagulation factors V, VIII, X, VII, XI, and **II,** etc.; converting plasminogen into plasmin; hydrolyzing complement, etc. Fibrinolysis inhibitors: including plasminogen activator inhibitor (PAI) and α2 antiplasmin (α2-AP). PAI mainly has two forms, PAI-1 and PAI-2, which may specifically bind to t-PA in a ratio of 1:1, thereby inactivating it and activating PLG at the same time. α2-AP is synthesized by the liver, and binds to PL in a ratio of 1:1 to form a complex to inhibit the activity of PL; FXIII makes α2-AP covalently bound to fibrin, reducing the sensitivity of fibrin to PL. Substances that inhibit the activity of the fibrinolytic system *in vivo:* PAI-1, complement C1 inhibitor; α2 antiplasmin; α2 macroglobulin.

The term "plasminogen pathway activator" herein encompasses a component of plasminogen activation pathway, a compound capable of directly activating plasminogen or indirectly activating plasminogen by activating a upstream component of plasminogen activation pathway, a compound simulating the activity of plasminogen or plasmin, a compound capable of upregulating the expression of plasminogen or an activator of plasminogen, a plasminogen analog, a plasmin analog, a tPA or uPA analog, and an antagonist of fibrinolysis inhibitor.

The term "component of plasminogen activation pathway" according to the present application encompasses:
1. plasminogen, Lys-plasminogen, Glu-plasminogen, micro-plasminogen, delta-plasminogen; variants or analogs thereof;
2. plasmin and a variant or analog thereof; and
3. plasminogen activators, such as tPA and uPA, and tPA or uPA variants and analogs comprising one or more domains of tPA or uPA, such as one or more kringle domains and proteolytic domains.

The term "an antagonist of a fibrinolysis inhibitor" encompasses an antagonist of PAI-1, complement C1 inhibitor, α2 antiplasmin or α2 macroglobulin, such as an antibody of PAI-1, complement C1 inhibitor, α2 antiplasmin or α2 macroglobulin.

"Variants" of the above plasminogen, plasmin, tPA and uPA include all naturally occurring human genetic variants as well as other mammalian forms of these proteins, as well as a protein obtained by addition, deletion and/or substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid, and still retaining the activity of plasminogen, plasmin, tPA or uPA. For example, "variants" of plasminogen, plasmin, tPA and uPA include mutational variants of these proteins obtained by substitution of such as 1-100, 1-90, 1-80, 1-70, 1-60, 1-50, 1- 45, 1-40, 1-35, 1-30, 1-25, 1-20, 1-15, 1-10, 1-5, 1-4, 1-3, 1-2, or 1 conservative amino acid.

A "plasminogen variant" of the present application encompasses a protein comprising or having at least 75%, 80%, 85%, 90%, 95%, 96%, 97%, 98% or 99% sequence identity with an amino acid sequence of SEQ ID NO: 2, 6, 8, 10 or 12, and retaining plasminogen activity and/or lysine binding activity. For example, a "plasminogen variant" according to the present application may be a protein obtained by addition, deletion and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid on the basis of SEQ ID NO: 2, 6, 8, 10 or 12, and still retaining plasminogen activity and/or lysine binding activity. Particularly, the plasminogen variants according to the present application include all naturally occurring human genetic variants as well as other mammalian forms of these proteins, as well as mutational variants of these proteins obtained by conservative amino acid substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid.

The plasminogen according to the present application may be a human plasminogen ortholog from a primate or rodent, or a variant thereof still retaining plasminogen activity and/or lysine binding activity, for example, a plasminogen represented by SEQ ID NO: 2, 6, 8, 10 or 12, such as a human natural plasminogen represented by SEQ ID NO: 2.

The "analogs" of the above plasminogen, plasmin, tPA, and uPA include compounds that respectively provide substantially similar effect to plasminogen, plasmin, tPA, or uPA.

The "variants" and "analogs" of above plasminogen, plasmin, tPA and uPA encompass "variants" and "analogs" of plasminogen, plasmin, tPA and uPA comprising one or more domains (e.g., one or more kringle domains and proteolytic domains). For example, "variants" and "analogs" of plasminogen encompass "variants" and "analogs" of plasminogen comprising one or more plasminogen domains (e.g., one or more kringle (k) domains and a proteolytic domain (or called as serine protease domain, or plasminogen protease domain)), such as mini-plasminogen. "Variants" and "analogs" of plasmin encompass "variants" and "analogs" of plasmin comprising one or more plasmin domains (e.g., one or more kringle domains and a proteolytic domain), such as mini-plasmin, and delta-plasmin.

Whether the above "variants" or "analogs" of plasminogen, plasmin, tPA or uPA respectively have the activity of plasminogen, plasmin, tPA or uPA, or whether the "variants" or "analogs" provide substantially similar effect to plasminogen, plasmin, tPA or uPA, may be detected by methods known in the art, for example, based on enzymography, ELISA (enzyme-linked immunosorbent assay), and FACS (fluorescence-activated cell sorting method), it is measured by the level of activated plasmin activity, for example, it is detected by referring to a method selected from the following documents: Ny, A., Leonardsson, G., Hagglund, A.C, Hagglof, P., Ploplis, VA., Carmeliet, P. and Ny, T. (1999). Ovulation inplasminogen-deficient mice. Endocrinology 140, 5030-5035; Silverstein RL, Leung LL, Harpel PC, Nachman RL (November 1984). "Complex formation of platelet thrombospondin with plasminogen. Modulation of activation by tissue

activator". J. Clin. Invest.74(5):1625-33; Gravanis I, Tsirka SE (February 2008). "Tissue-type plasminogen activator as a therapeutic target in stroke". Expert Opinion on Therapeutic Targets. 12(2):159-70; Geiger M, Huber K, Wojta J, Stingl L, Espana F, Griffin JH, Binder BR (Aug 1989). "Complex formation between urokinase and plasma protein C inhibitor in vitro and in vivo". Blood. 74(2):722-8.

In some embodiments of the present application, the "component of plasminogen activation pathway" according to the present application is a plasminogen selected from the group consisting of: Glu-plasminogen, Lys-plasminogen, mini-plasminogen, micro-plasminogen, delta-plasminogen, or variants thereof retaining plasminogen activity. In some embodiments, the plasminogen is natural or synthetic human plasminogen, or a conservative mutant variant or fragment thereof still retaining plasminogen activity and/or lysine binding activity. In some embodiments, the plasminogen is a human plasminogen ortholog from a primate or rodent or a conservative mutant variant or fragment thereof still retaining plasminogen activity and/or lysine binding activity. In some embodiments, the amino acid sequence of the plasminogen comprises or has an amino acid sequence represented by SEQ ID NO: 2, 6, 8, 10 or 12. In some embodiments, the plasminogen is a human full length plasminogen. In some embodiments, the plasminogen is a human full length plasminogen represented by SEQ ID NO: 2.

"A compound capable of directly activating plasminogen, or indirectly activating plasminogen by activating a upstream component of plasminogen activation pathway", refers to any compound capable of directly activating plasminogen, or indirectly activating plasminogen by activating a upstream component of plasminogen activation pathway, such as tPA, uPA, streptokinase, saruplase, alteplase, reteplase, tenecteplase, anistreplase, monteplase, lanoteplase, pamiteplase, staphylokinase.

The "antagonist of a fibrinolysis inhibitor" according to the present application is a compound that antagonizes, weakens, blocks, or prevents the action of a fibrinolysis inhibitor. Such fibrinolysis inhibitors are e.g., PAI-1, complement C1 inhibitor, α2 antiplasmin, and α2 macroglobulin. Such an antagonist is: e.g., an antibody of PAI-1, complement C1 inhibitor, α2 antiplasmin, or α2 macroglobulin; or an antisense RNA or small RNA blocking or downregulating the expression of such as PAI-1, complement C1 inhibitor, α2 antiplasmin or α2 macroglobulin; or a compound occupying the binding site of PAI-1, complement C1 inhibitor, α2 antiplasmin, or α2 macroglobulin but without the function of PAI-1, complement C1 inhibitor, α2 antiplasmin, or α2 macroglobulin; or a compound blocking the binding and/or active domains of PAI-1, complement C1 inhibitor, α2 antiplasmin, or α2 macroglobulin.

Plasmin is a key component of the plasminogen activation system (PA system). It is a broad-spectrum protease capable of hydrolyzing several components of the extracellular matrix (ECM), including fibrin, gelatin, fibronectin, laminin, and proteoglycans. In addition, plasmin may activate some metalloproteinase precursors (pro-MMPs) to form active metalloproteinases (MMPs). Therefore, plasmin is considered to be an important upstream regulator of extracellular proteolysis. Plasmin is formed by proteolysis of plasminogen by two physiological PAs: tissue-type plasminogen activator (tPA) or urokinase-type plasminogen activator (uPA). Due to the relatively high levels of plasminogen in plasma and other body fluids, it has traditionally been thought that the regulation of the PA system is mainly achieved through the synthesis and activity levels of PAs. The synthesis of components of PA system is strictly regulated by different factors, such as hormone, growth factor and cytokine. In addition, there are specific physiological inhibitors of plasmin and PAs. The main inhibitor of plasmin is α2-antiplasmin. The activity of PAs is inhibited by plasminogen activator inhibitor-1 (PAI-1) of both uPA and tPA, and regulated by plasminogen activator inhibitor-2 (PAI-2) which mainly inhibits uPA. Certain cell surfaces have uPA-specific cell surface receptors (uPARs) with direct hydrolytic activity.

Plasminogen is a single-chain glycoprotein consisting of 791 amino acids with a molecular weight of approximately 92 kDa. Plasminogen is mainly synthesized in the liver, and is abundantly present in the extracellular fluid. The content of plasminogen in plasma is approximately 2 µM. Plasminogen is thus a huge potential source of proteolytic activity in tissues and body fluids. Plasminogen exists in two molecular forms: glutamate-plasminogen (Glu-plasminogen) and lysine-plasminogen (Lys-plasminogen). The naturally secreted and uncleaved form of plasminogen has an amino-terminal (N-terminal) glutamate, and is therefore referred to as glutamate-plasminogen. However, in the presence of plasmin, glutamate-plasminogen is hydrolyzed at Lys76-Lys77 into lysine-plasminogen. Compared with glutamate-plasminogen, lysine-plasminogen has a higher affinity for fibrin, and may be activated by PAs at a higher rate. The Arg560-Val561 peptide bond of these two forms of plasminogen may be cleaved by either uPA or tPA, resulting in the formation of two-chain protease plasmin linked by disulfide. The amino-terminal part of plasminogen comprises five homologous tri-cycles, i.e., so-called kringles, and the carboxy-terminal part comprises the protease domain. Some kringles comprise lysine-binding sites that mediate the specific interaction of plasminogen with fibrin and its inhibitor α2-AP. A recently found plasminogen is a 38 kDa fragment, comprising kringles1-4, and it is a potent inhibitor of angiogenesis. This fragment is named as angiostatin, and is produced by the hydrolysis of plasminogen by several proteases.

The main substrate of plasmin is fibrin, and the dissolution of fibrin is the key to preventing pathological thrombosis. Plasmin also has substrate specificity for several components of the ECM, including laminin, fibronectin, proteoglycans, and gelatin, indicating that plasmin also plays an important role in ECM remodeling. Indirectly, plasmin may also degrade other components of the ECM, including MMP-1, MMP-2, MMP-3 and MMP-9, by converting certain protease precursors into active proteases. Therefore, it has been proposed that plasmin may be an important upstream regulator of extracellular proteolysis. In addition, plasmin has the ability to activate certain latent forms of growth factors. In vitro, plasmin also hydrolyzes components of the complement system, and releases chemotactic complement fragments.

"Plasmin" is a very important enzyme present in the blood that hydrolyzes fibrin clots into fibrin degradation products and D-dimers.

"Plasminogen" is the zymogen form of plasmin. According to the sequence in swiss prot, it consists of 810 amino acids calculating by the natural human plasminogen amino acid sequence (SEQ ID NO: 4) containing the signal peptide, and the molecular weight is about 90kD, and it is a glycoprotein mainly synthesized in the liver and capable of circulating in the blood, the cDNA sequence encoding this amino acid sequence is represented by SEQ ID NO: 3. Full-length plasminogen comprises seven domains: a C-terminal serine protease domain, an N-terminal Pan Apple (PAp) domain, and five Kringle domains (Kringle1-5). Referring to the sequence in swiss prot, its signal peptide comprises residues Met1-Gly19, PAp comprises residues Glu20-Val98, Kringle1 comprises residues Cys103-Cys181, Kringle2 comprises residues Glu184-Cys262, Kringle3 comprises residues Cys275-Cys352, Kringle4 comprises residues Cys377-Cys454, and Kringle5 comprises residues Cys481-Cys560. According to NCBI data, the serine protease domain comprises residues Val581-Arg804.

Glu-plasminogen is a natural full-length plasminogen, consisting of 791 amino acids (without a signal peptide of 19 amino acids); the cDNA sequence encoding this amino acid sequence is represented by SEQ ID NO: 1, and the amino acid sequence is represented by SEQ ID NO: 2. *In vivo,* there is also a Lys-plasminogen produced by the hydrolysis of the peptide bond between amino acids 76 and 77 of Glu-plasminogen, as represented by SEQ ID NO: 6; and the cDNA sequence encoding this amino acid sequence is represented by SEQ ID NO: 5. Delta-plasminogen (δ-plasminogen) is a fragment of full-length plasminogen that lacks the Kringle2-Kringle5 structure, and only comprises Kringle1 and a serine protease domain (also known as a proteolysis domain, or a plasminogen protease domain). The amino acid sequence of delta-plasminogen (SEQ ID NO: 8) is reported in a literature, and the cDNA sequence encoding this amino acid sequence is represented by SEQ ID NO: 7. Mini-plasminogen consists of Kringle5 and a serine protease domain, and it is reported that it comprises residues Val443-Asn791 (with the Glu residue of the Glu-plasminogen sequence without the signal peptide as the starting amino acid), its amino acid sequence is represented by SEQ ID NO: 10, and the cDNA sequence encoding this amino acid sequence is represented by SEQ ID NO: 9. While micro-plasminogen comprises only a serine protease domain, and it is reported that its amino acid sequence comprises residues Ala543-Asn791 (with the Glu residue of the Glu-plasminogen sequence without the signal peptide as the starting amino acid); additionally, it is disclosed in patent document CN102154253A that its sequence comprises residues Lys531-Asn791 (with the Glu residue of the Glu-plasminogen sequence without the signal peptide as the starting amino acid); the sequence of the present patent application refers to the patent document CN102154253A, the amino acid sequence is represented by SEQ ID NO: 12, and the cDNA sequence encoding this amino acid sequence is represented by SEQ ID NO: 11.

In the present application, "plasmin" and "fibrinolytic enzyme" may be used interchangeably with the same meaning; "plasminogen" and "fibrinolytic zymogen" may be used interchangeably with the same meaning.

In the present application, "lack" of plasminogen or plasminogen activity means that the content of plasminogen in a subject is lower than that of a normal person, and is sufficiently low to affect the normal physiological function of the subject; "deficiency" of plasminogen or plasminogen activity means that the content of plasminogen in a subject is significantly lower than that of a normal person, and even the activity or expression is extremely low, and the normal physiological function may only be maintained by external supply of plasminogen.

Those skilled in the art may understand that, all technical solutions of plasminogen according to the present application are applicable to plasmin, thus the technical solutions described in the present application encompass plasminogen and plasmin. During circulation, plasminogen is present in a closed, inactive conformation, but when bound to a thrombus or cell surface, it is converted into active plasmin with an open conformation after being mediated by plasminogen activator (PA). Active plasmin may further hydrolyze the fibrin clot into degradation products of fibrin and D-dimers, thereby dissolving the thrombus. The PAp domain of plasminogen comprises an important determinant for maintaining plasminogen in an inactive closed conformation, while the KR domain may bind to a lysine residue present on a receptor and substrate. A variety of enzymes are known to act as plasminogen activators, including: tissue plasminogen activator (tPA), urokinase plasminogen activator (uPA), kallikrein, and coagulation factor XII (Hageman factor) etc.

"Active fragments of plasminogen" in this application include 1) an active fragment in plasminogen protein that is capable of binding to a target sequence in substrates, also known as lysine-binding fragment, for example a fragment comprising Kringle1, Kringle 2, Kringle 3, Kringle 4 and/or Kringle 5 (for the structure of the plasminogen, see Aisina RB, Mukhametova L I. Structure and function of plasminogen/plasmin system [J]. Russian Journal of Bioorganic Chemistry, 2014,40(6):590-605); 2) an active fragment that plays a proteolytic function in the plasminogen protein, e.g., a fragment with the plasminogen activity (proteolytic function) represented by SEQ ID NO: 14; 3) a fragment in the plasminogen protein that has both the binding activity to the target sequence in the substrate (lysine binding activity) and the plasminogen activity (proteolytic function). In some embodiments of the present application, the plasminogen is a protein comprising the active fragment of plasminogen represented by SEQ ID NO: 14. In some embodiments of the present application, the plasminogen is a protein comprising a lysine-binding fragment of Kringle 1, Kringle 2, Kringle 3, Kringle 4 and/or Kringle 5. In some embodiments, the plasminogen active fragment of the present application comprises SEQ ID NO: 14, or an amino acid sequence having at least 80%, 90%, 95%, 96%, 97%, 98%, 99% homology with SEQ ID NO: 14. Therefore, the plasminogen of the present application comprises a protein comprising the active fragment of the plasminogen and still maintaining the activity of the plasminogen. In some embodiments, the plasminogen of the present application comprises Kringle 1, Kringle 2, Kringle 3, Kringle 4 and/or Kringle 5, or a protein has at least 80%, 90%, 95%, 96%, 97%, 98%, 99% homologous with Kringle 1, Kringle 2, Kringle 3, Kringle 4 or Kringle 5 and still have lysine-binding activity.

At present, the methods for measuring plasminogen and its activity in blood comprise: detection of tissue plasminogen activator activity (t-PAA), detection of plasma tissue plasminogen activator antigen (t-PAAg), detection of plasma tissue plasminogen activity (plgA), detection of plasma tissue plasminogen antigen (plgAg), detection of the activity of plasma tissue plasminogen activator inhibitor, detection of the antigen of plasma tissue plasminogen activator inhibitor, and detection of plasma plasmin-antiplasmin complex (PAP); wherein the most commonly used detection method is the chromogenic substrate method: adding streptokinase (SK) and a chromogenic substrate to the plasma to be detected, the PLG in the plasma to be detected is converted into PLM under the action of SK, and PLM acts on the chromogenic substrate; subsequently, the detection by spectrophotometer indicates that the increase in absorbance is directly proportional to plasminogen activity. In addition, the plasminogen activity in blood may also be detected by immunochemical method, gel electrophoresis, immunoturbidimetry, and radioimmunodiffusion method.

"Ortholog or orthologs" refer to homologs between different species, including both protein homologs and DNA homologs, also known as orthologs and vertical homologs; particularly it refers to proteins or genes evolved from the same ancestral gene in different species. The plasminogen according to the present application includes human natural plasminogen, and further includes plasminogen ortholog or orthologs derived from different species and having plasminogen activity.

A "conservative substitution variant" refers to a variant in which a given amino acid residue is altered without changing the overall conformation and function of the protein or enzyme, including but not limited to those variants in which the amino acid(s) in the amino acid sequence of the parent protein are replaced by amino acid(s) with similar properties (e.g., acidic, basic, hydrophobic, etc.). Amino acids with similar properties are well known in the art. For example, arginine, histidine and lysine are hydrophilic basic amino acids and are interchangeable. Similarly, isoleucine is a hydrophobic amino acid, and may be replaced by leucine, methionine or valine. Therefore, the similarity of two proteins or amino acid sequences with similar functions may differ; for example, 70% to 99% similarity (identity) based on the MEGALIGN algorithm. "Conservative substitution variants" also include polypeptides or enzymes having more than 60%, preferably more than 75%, more preferably more than 85%, or even most preferably more than 90% amino acid identity determined by BLAST or FASTA algorithm, and having the same or substantially similar properties or functions as the natural or parent protein or enzyme.

"Pathological TDP-43 protein" is a term contrasted with "physiologically functional TDP-43 protein". The physiologically functional TDP-43 protein refers to a TDP-43 protein that is in a state where it can exhibit its desired function in the *in vivo* cellular environment. In contrast, a "pathological TDP-43 protein" refers to a TDP-43 protein that is unable to perform its desired function in an *in vivo* cellular environment. Examples of pathological TDP-43 proteins include, but are not limited to, TDP-43 proteins that have mutated and lost their physiological functions (e.g., lost more than 50%, 60%, 70%, 80%, 90% of their relevant physiological functions), TDP-43 proteins that form protein aggregates, misfolded TDP-43 proteins, abnormally modified TDP-43 proteins (including hyperphosphorylation, ubiquitination, acetylation, and C-terminal fragments cleaved by proteolysis), and TDP-43 proteins that have undergone protein denaturation. Another characteristic feature of TDP-43 pathological conditions is the redistribution and accumulation of TDP-43 from the nucleus to the cytoplasm, and such proteins are also included in the scope of the pathological TDP-43 proteins of the present application. In the present application, in the context of plasminogen "promotes TDP-43 (protein) degradation", it means that plasminogen promotes the degradation of pathological TDP-43 (protein).

"Isolated" plasminogen refers to a plasminogen protein isolated and/or recovered from its natural environment. In some embodiments, the plasminogen will be purified: (1) to more than 90%, more than 95%, or more than 98% purity (by weight), as determined by Lowry's method, e.g., more than 99% (by weight), (2) to a degree sufficient to obtain at least 15 residues of the N-terminal or internal amino acid sequence by using a spinning cup sequence analyzer, or (3) to homogeneity as determined by using Coomassie blue or silver staining through sodium dodecyl sulfate-polyacrylamide gel electrophoresis (SDS-PAGE) under reducing or non-reducing conditions. Isolated plasminogen also includes plasminogen prepared from recombinant cells by bioengineering techniques and isolated by at least one purification step.

The terms "polypeptide", "peptide" and "protein" are used interchangeably herein to refer to a polymeric form of amino acids of any length, which may include genetically encoded and non-genetically encoded amino acids, chemically or biochemically modified or derivatized amino acids, and polypeptides with modified peptide backbones. The terms include fusion proteins including, but not limited to, fusion proteins with heterologous amino acid sequences, fusions with heterologous and homologous leader sequences (with or without N-terminal methionine residues); and the like.

"Percent (%) of amino acid sequence identity" with respect to a reference polypeptide sequence is defined as, after introducing gaps as necessary to achieve maximum percent sequence identity, and no conservative substitutions are considered as part of the sequence identity, the percentage of amino acid residues in a candidate sequence that are identical to the amino acid residues in a reference polypeptide sequence. Alignment for purposes of determining percent amino acid sequence identity may be accomplished in a variety of ways within the technical scope in the art, e.g., by publicly available computer software, such as BLAST, BLAST-2, ALIGN or Megalign (DNASTAR) software. Those skilled in the art can determine the appropriate parameters for aligning sequences, including any algorithms needed to achieve maximal alignment for the full length of the sequences to be compared. However, for the purpose of the present application, the values of percent of amino acid sequence identity are generated by using the computer program ALIGN-2 for sequence comparison.

Where ALIGN-2 is used to compare amino acid sequences, the percentage (%) of amino acid sequence identity of a given amino acid sequence A relative to a given amino acid sequence B (or may be expressed as a given amino acid sequence A having a certain percentage (%) of amino acid sequence identity relative to, with or with respective to a given amino acid sequence B) is calculated as follows:
Fraction X/Y times 100; wherein X is the number of amino acid residues scored as identical matches during the alignment of sequences A and B by the sequence alignment program ALIGN-2, and wherein Y is the total number of amino acid residues in sequence B. It should be appreciated that, where the length of amino acid sequence A is not equal to that of amino acid sequence B, the percentage (%) of amino acid sequence identity of A with respect to B will not equal to the percentage (%) of amino acid sequence identity of B with respect to A. Unless expressly stated otherwise, all the values of percentage (%) of amino acid sequence identity used herein are obtained by using the ALIGN-2 computer program as described in the preceding paragraph.

The terms "individual", "subject" and "patient" are used interchangeably herein to refer to mammals including, but not limited to, murine (rat, mouse), non-human primate, human, canine, feline, hoofed animals (e.g., horses, cattle, sheep, pigs, goats), etc.

A "therapeutically effective amount" or "effective amount" refers to an amount of plasminogen sufficient to prevent and/or treat a disease when administrated to a mammal or other subject for treating the disease. A "therapeutically effective amount" will vary depending on the plasminogen used, the severity of the disease and/or symptoms in the subject to be treated, as well as the age, weight, and the like.

The term "treatment" of a disease state includes inhibiting or preventing the development of the disease state or its clinical symptoms, or alleviating the disease state or symptoms, resulting in temporary or permanent regression of the disease state or its clinical symptoms.

### Preparation of the Plasminogen According to the Present Application

Plasminogen may be isolated from nature, and purified for further therapeutic use, or it may be synthesized by standard chemical peptide synthesis techniques. When the polypeptide is synthesized chemically, the synthesis may be carried out via liquid phase or solid phase. Solid-phase polypeptide synthesis (SPPS) (in which the C-terminal amino acid of the sequence is attached to an insoluble support, followed by the sequential addition of the retaining amino acids in the sequence) is a suitable method for chemical synthesis of plasminogen. Various forms of SPPS, such as Fmoc and Boc, may be used to synthesize plasminogen. Techniques for solid-phase synthesis are described in Barany and Solid-Phase Peptide Synthesis; pp.3-284 in The Peptides: Analysis, Synthesis, Biology. Vol. 2: Special Methods in Peptide Synthesis, Part A., Merrifield, et al. J. Am. Chem. Soc., 85:2149-2156 (1963); Stewart et al., Solid Phase Peptide Synthesis, 2nd ed. Pierce Chem. Co., Rockford, Ill. (1984); and Ganesan A. 2006 Mini Rev. Med Chem. 6:3-10, and Camarero JA et al. 2005, Protein Pept Lett. 12:723-8. Briefly, small insoluble porous beads are treated with functional units on which peptide chains are constructed; after repeated cycles of coupling/deprotection, the attached solid-phase free N-terminal amine is coupled to a single N-protected amino acid unit. This unit is then deprotected to reveal new N-terminal amines that may be attached to other amino acids. The peptide remains immobilized on the solid phase, subsequently it is cleaved off.

Plasminogen according to the present application may be produced by standard recombinant methods. For example, a nucleic acid encoding plasminogen is inserted into an expression vector to operably link to regulatory sequences in the expression vector. The regulatory sequences for expression include, but are not limited to, promoters (e.g., naturally associated or heterologous promoters), signal sequences, enhancer elements, and transcription termination sequences. Expression regulation may be a eukaryotic promoter system in a vector capable of transforming or transfecting a eukaryotic host cell (e.g., COS or CHO cell). Once the vector is incorporated into a suitable host, the host is maintained under conditions suitable for high-level expression of the nucleotide sequence and collection and purification of plasminogen.

A suitable expression vector typically replicates in a host organism as an episome or as an incorporated part of the host chromosomal DNA. Typically, an expression vector contains a selectable marker (e.g., ampicillin resistance, hygromycin resistance, tetracycline resistance, kanamycin resistance, or neomycin resistance marker) to facilitate the detection of those cells transformed with desired exogenous DNA sequence.

*Escherichia coli* is an example of a prokaryotic host cell that may be used to clone a plasminogen-encoding polynucleotide. Other microbial hosts suitable for use include bacilli such as *Bacillus subtilis,* and other *enterobacteriaceae* such as *Salmonella, Serratia,* and various *Pseudomonas* species. In these prokaryotic hosts, expression vectors may also be generated, which will typically comprise an expression control sequence (e.g., origin of replication) that are compatible with the host cell. In addition, there are many well-known promoters, such as the lactose promoter system, the tryptophan (trp) promoter system, the beta-lactamase promoter system, or the promoter system from bacteriophage lambda. A promoter will typically control the expression, optionally in case of an operator gene sequence, and have ribosome binding site sequence, etc., to initiate and complete transcription and translation.

Other microorganisms, such as yeast, may also be used for expression. Yeast (e.g., S. *cerevisiae)* and *Pichia* are examples of suitable yeast host cells, and as required a suitable vector has an expression control sequence (e.g., promoter), origin of replication, termination sequence, etc. A typical promoter comprises 3-phosphoglycerate kinase and other saccharolytic enzymes. Particularly, inducible yeast promoters include promoters from ethanol dehydrogenase, isocytochrome C, and enzymes responsible for maltose and galactose utilization.

In addition to microorganisms, mammalian cells (e.g., mammalian cells grown in *in vitro* cell culture) may also be used to express and produce the plasminogen of the application (e.g., polynucleotides encoding the plasminogen). See Winnacker, From Genes to Clones, VCH Publishers, N.Y., N.Y. (1987). Suitable mammalian host cells include CHO cell lines, various Cos cell lines, HeLa cells, myeloma cell lines, and transformed B cells or hybridomas. Expression vectors for use in these cells may comprise expression control sequences such as origin of replication, promoter and enhancer (Queen et al., Immunol. Rev. 89:49 (1986)), and necessary sites for processing information such as ribosome binding sites, RNA splicing sites, polyadenylation sites, and transcription terminator sequences. Examples of suitable expression control sequences are promoters derived from immunoglobulin gene, SV40, adenovirus, bovine papilloma virus, cytomegalovirus, and the like. See Co et al, J. Immunol. 148:1149 (1992).

Once synthesized (chemically or recombinantly), the plasminogen of the present application may be purified according to standard procedures in the art, including ammonium sulfate precipitation, affinity column, column chromatography, high performance liquid chromatography (HPLC), gel electrophoresis, and the like. The plasminogen is substantially pure, e.g., at least about 80-85% pure, at least about 85-90% pure, at least about 90-95% pure, or 98-99% pure or purer, e.g., free of contaminants such as cellular debris, macromolecules other than the plasminogen, and the like.

### Medicament Formulation

A therapeutic formulation may be prepared by mixing the plasminogen of desired purity with an optional pharmaceutical carrier, excipient, or stabilizer (Remington's Pharmaceutical Sciences, 16th edition, Osol, A. ed. (1980)), to form a lyophilized formulation or aqueous solution. An acceptable carrier, excipient, or stabilizer is non-toxic to a recipient at the dosage and concentration used, including buffers such as phosphate, citrate and other organic acids; antioxidants such as ascorbic acid and methionine; preservatives (such as octadecyl dimethyl benzyl ammonium chloride; hexane diamine chloride; benzalkonium chloride, benzethonium chloride; phenol, butanol or benzyl alcohol; alkyl parabens such as methyl or propyl paraben; catechol; resorcinol; cyclohexanol; 3-pentanol; m-cresol); low molecular weight polypeptides (less than about 10 residues); proteins such as serum albumin, gelatin or immunoglobulins; hydrophilic polymers such as polyvinylpyrrolidone; amino acids such as glycine, glutamine, asparagine, histidine, arginine or lysine; monosaccharides, disaccharides and other carbohydrates such as glucose, mannose, or dextrin; chelating agents such as EDTA; carbohydrates such as sucrose, mannitol, fucose, or sorbitol; salt-forming counterions such as sodium; metal complexes (such as zinc-protein complexes); and/or nonionic surfactants such as TWENTM, PLURONICSTM or polyethylene glycol (PEG).

The formulations according to the present application may further comprise one or more active compounds as required for the particular condition to be treated, preferably those compounds are complementary in activity and do not have side effects on each other. For example, antihypertensive medicaments, antiarrhythmic medicaments, medicaments for treating diabetes, etc.

The plasminogen according to the present application may be encapsulated in microcapsules prepared by techniques such as coacervation or interfacial polymerization, for example, the plasminogen may be placed in colloidal drug delivery systems (e.g., liposomes, albumin microspheres, microemulsions, nanoparticles and nanocapsules) or in hydroxymethyl cellulose or gel-microcapsules and poly-(methyl methacrylate) microcapsules in macroemulsions. These techniques are disclosed in Remington's Pharmaceutical Sciences 16th edition, Osol, A. Ed. (1980).

The plasminogen according to the present application for *in vivo* administration must be sterile. This may be easily achieved by filtration through sterilizing filters before or after lyophilization and reformulation.

The plasminogen according to the present application may be prepared as a sustained-release formulation. Suitable examples of sustained-release formulations include semipermeable matrices of solid hydrophobic polymers which have a certain shape and contain glycoprotein, for example, membranes or microcapsules. Examples of sustained-release matrices include polyesters, hydrogels such as poly(2-hydroxyethyl-methacrylate) (Langer et al., J. Biomed. Mater. Res., 15:167-277

(1981); Langer, Chem. Tech., 12:98-105 (1982)), or poly(vinyl alcohol), polylactide (US Pat. No.3,773,919, EP58,481), copolymers of L-glutamic acid and γ-ethyl-L-glutamic acid (Sidman, et al., Biopolymers 22:547 (1983)), non-degradable ethylene-vinyl acetate (Langer, et al., supra), or degradable lactic acid-glycolic acid copolymers such as Lupron Depot^{™} (injectable microspheres consisting of lactic acid-glycolic acid copolymer and leuprolide acetate), and poly-D-(-)-3-hydroxybutyric acid. Polymers such as ethylene-vinyl acetate and lactic acid-glycolic acid may release molecules continuously for more than 100 days, while some hydrogels release proteins for shorter period of time. Rational strategies to stabilize proteins may be devised based on the relevant mechanisms. For example, if the mechanism of condensation is found to form intermolecular S-S bond through thiodisulfide bond interchange, then stabilization may be achieved by modifying sulfhydryl residues, lyophilizing from acidic solutions, controlling humidity, using suitable additives, and developing specific polymer matrix composition.

### Administration and Dosage

Administration of the pharmaceutical composition according to the present application may be accomplished by different means, e.g., intravenously, intraperitoneally, subcutaneously, intracranially, intrathecally, intraarterally (e.g., via the carotid artery), and intramuscularly.

Preparations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, or fixed oils. Intravenous vehicles include fluid and nutritional supplements, electrolyte supplements, and the like. Preservatives and other additives may also be present, for example, antimicrobials, antioxidants, chelating agents, and inert gases, etc.

Dosing regimens will be determined by medical personnel based on various clinical factors. As is well known in the medical field, the dosage for any patient depends on a variety of factors, including the patient's size, body surface area, age, the particular compound to be administrated, sex, number and route of administration, general health, and other concomitantly administrated medicaments. The dosage range of the pharmaceutical composition comprising the plasminogen according to the present application may be about 0.0001-2000 mg/kg, or about 0.001-500 mg/kg (e.g., 0.02 mg/kg, 0.25 mg/kg, 0.5 mg/kg, 0.75 mg/kg, 10 mg/kg, 50 mg/kg, etc.) body weight of the subject per day. For example, the dose may be 1 mg/kg body weight, or 50 mg/kg body weight, or in the range of 1-50 mg/kg, or at least 1 mg/kg. Dosages above or below this exemplary range are also contemplated, especially in view of the factors set forth above. Intermediate doses within the above ranges are also included within the scope of the present application. Subjects may be administrated such doses daily, every other day, weekly, or according to any other schedule determined by empirical analysis. An exemplary dosage schedule includes 1-10 mg/kg on consecutive days. Real-time evaluation of therapeutic efficacy and safety is required during the administration of the medicament of the present application.

### Product or Kit

One embodiment of the present application relates to a product or kit comprising the plasminogen or plasmin according to the present application for treating cardiovascular disease caused by diabetes and related disorders. The product preferably comprises a container, and a label or package insert. Suitable containers are bottles, vials, syringes, etc. The container may be made of various materials such as glass or plastic. The container contains a composition which is effective for treatment of the disease or disorder according to the present application and has a sterile access port (e.g., the container may be an intravenous solution pack or vial containing a stopper penetrable by a hypodermic needle). At least one active agent in the composition is plasminogen/plasmin. The label on or attached to the container indicates that the composition is used for treatment of cardiovascular disease caused by diabetes and related disorders according to the present application. The product may further comprise a second container containing a pharmaceutically acceptable buffer, such as phosphate buffered saline, Ringer's solution, and dextrose solution. It may further contain other materials required from a commercial and user standpoint, including other buffers, diluents, filters, needles and syringes. In addition, the product comprises a package insert with instructions for use, including, for example, instructing the user of the composition to administrate the plasminogen composition to the patient along with other medicaments for treatment of concomitant diseases.

The term "muscle atrophy" as used herein refers to a reduction in the amount, structural abnormality, or reduction or loss, and/or functional abnormality, or weakening or loss of muscle tissue due to various reasons. The main causes of muscular atrophy are various muscle diseases or injuries, including syringomyelia, myelitis, radiculopathic spondyloarthritis, basal arachnoiditis, brainstem lesions, and brain and spinal nerve lesions.

### EXAMPLES

The human plasminogen used in all the following examples was from donor plasma, based on the methods described in the following literatures to optimize: Kenneth C Robbins, Louis Summaria, David Elwyn et al. Further Studies on the Purification and Characterization of Human Plasminogen and Plasmin. Journal of Biological Chemistry,1965,240(1):541-550; Summaria L, Spitz F, Arzadon L et al. Isolation and characterization of the affinity chromatography forms of human Glu-and Lys-plasminogens and plasmins. J Biol Chem. 1976 Jun 25; 251(12):3693-9; HAGAN JJ, ABLONDI FB, DE RENZO EC. Purification and Biochemical Properties of Human Plasminogen. J Biol Chem. 1960 Apr; 235: 1005-10 [1-3], and the plasminogen was purified from human donor plasma, in which human Lys-plasminogen and Glu-plasminogen are >98%.

### EXAMPLES

### Example 1: Plasminogen Promotes the Cleavage of Pathological TDP-43 Protein in Normal Mouse Brain Homogenate

Four C57BL/6J male mice aged 11-12 weeks and weighing 18-25 g were sacrificed, and the whole brain was taken out to weigh. 1×PBS was added at 150 mg tissue/mL PBS (Thermo Fisher, pH 7.4; 10010-031) to homogenize at 4°C (1 min, 3-4 times). After homogenization, centrifugation was performed at 4°C (12000 rpm, 20 min), and the supernatant, i.e., the brain homogenate, was placed in a new EP tube.

Eppendorf (EP) tubes were set as: ① blank group, ② blank control group, vehicle control group, and ④ plasminogen group, with 5 parallels in each group. The blank group was added with 21.5 µL of normal saline, 4.6 µL of vehicle solution (10 mM sodium citrate, 2% arginine hydrochloride, 3% mannitol, pH 7.4), and 23.9 µL of mouse brain homogenate; the blank control group was added with 21.5 µL of normal saline, 2.3 µL of plasminogen solution (2 mg/mL), and 23.9 µL of mouse brain homogenate; the vehicle control group was added with 20.5 µL of TDP-43 (Nanjing GenScript Biotechnology Co., Ltd., custom-expressed human TDP-43, C134WHE160-2/PSHF001, 1.05 mg/mL), 4.6 µL of vehicle solution, and 23.9 µL of mouse brain homogenate; the plasminogen group was added with 20.5 µL of TDP-43 (1.05 mg/mL), 2.3 µL of plasminogen solution (2 mg/mL), and 23.9 µL of mouse brain homogenate. After adding each group of samples, they are incubated at 37°C for 3 h, then adding 50 µL of 0.1% trifluoroacetic acid solution to terminate the reaction.

A 12% gel was prepared according to the SDS-PAGE gel preparation instructions. Each group of samples was mixed with 4× loading buffer (TaKaRa, e2139) at a volume ratio of 3:1, heating at 100°C for 5 min, cooling and centrifuging for 2 min, and then 20 µL was taken for sample loading. The electrophoresis conditions are as follows: running at 30 V for 45 min, then 100 V to the bottom of the gel. After electrophoresis, the gel was stripped and transferred to an activated PVDF membrane (GE, A29433753) under the electrophoresis conditions of 15 V for 2.5 h. After transferring, the PVDF membrane was immersed in blocking solution (5% skim emulsion) to block overnight in a 4°C refrigerator. After washing 4 times with TBST (0.01M Tris-NaCl, pH 7.6 buffer), rabbit anti-human TDP-43 antibody (Proteintech (China), 12892-1-AP) and cyclophilin antibody were added to incubate at room temperature for 1.5 h. After washing 4 times with TBST, goat anti-rabbit IgG (HRP) antibody (Abcam, ab6721) secondary antibody was added to incubate at room temperature for 1 h. After washing 4 times with TBST, the PVDF membrane was placed on a clean imaging plate, Immobilon Western HRP Substrate (MILLIPORE, WBKLS0100) was added for color development, and photos were taken under a biomolecular imager, then performing quantitative analysis by using Image J.

The results show that, the molecular weight of the recombinant TDP-43 protein monomer is about 43 kDa, the molecular weight of the high molecular weight TDP-43 protein (HMW) is >55 kDa, and the molecular weight of the low molecular weight TDP-43 fragment (LMW) is <40 kDa. In addition, in the normal mouse brain homogenate, the amounts of TDP-43 monomer, HMW and LMW in the plasminogen group are significantly lower than those in the vehicle control group, and the difference is extremely significant (*** represents P < 0.001, * represents P < 0.05) (Fig. 2). This suggests that plasminogen can promote the cleavage of TDP-43 in normal mouse brain homogenate.

### Example 2: Plasminogen Promotes the Cleavage of Pathological TDP-43 Protein in Brain Homogenate of Amyotrophic Sclerosis Model Mice

Four B6.Cg-Tg (SOD1-G93A)1Gur/J transgenic male mice (referred to as SOD1-G93A transgenic mice) were sacrificed, and the whole brain was taken out to weighe. 1×PBS was added at 150 mg tissue/mL PBS (Thermo Fisher, pH 7.4; 10010-031) to homogenize at 4°C (1 min, 3-4 times). After homogenization, the supernatant, i.e., the brain homogenate was taken out to place in a new EP tube.

Eppendorf (EP) tubes were set as: ① blank group, ② blank control group, ③ vehicle control group, and ④ plasminogen group, with 5 parallels in each group. The blank group was added with 21.5 µL of normal saline, 4.6 µL of vehicle solution (10 mM sodium citrate, 2% arginine hydrochloride, 3% mannitol, pH 7.4), and 23.9 µL of mouse brain homogenate; the blank control group was added with 21.5 µL of normal saline, 2.3 µL of plasminogen solution (2 mg/mL), and 23.9 µL of mouse brain homogenate; the vehicle control group was added with 20.5 µL of TDP-43 (Nanjing GenScript Biotechnology Co., Ltd., custom-expressed human TDP-43, C134WHE160-2/PSHF001, 1.05 mg/mL), 4.6 µL of vehicle solution, and 23.9 µL of mouse brain homogenate; the plasminogen group was added with 20.5 µL of TDP-43 (1.05 mg/mL), 2.3 µL of plasminogen solution (2 mg/mL), and 23.9 µL of mouse brain homogenate. After adding the samples of each group, they were incubated at 37°C for 3 h, and then 50 µL of 0.1% trifluoroacetic acid solution was added to terminate the reaction.

A 12% gel was prepared according to the SDS-PAGE gel preparation instructions. Each group of samples was mixed with 4× loading buffer (TaKaRa, e2139) at a volume ratio of 3:1, heating at 100°C for 5 min, cooling and centrifuging for 2 min, and then 20 µL was taken for sample loading. The electrophoresis conditions are as follows: running at 30 V for 45 min, then 100 V to the bottom of the gel. After electrophoresis, the gel was stripped and transferred to an activated PVDF membrane (GE, A29433753) under the conditions of 15 V for 2.5 h. After transferring, the PVDF membrane was immersed in blocking solution (5% skim emulsion) to block overnight in a 4°C refrigerator. After washing 4 times with TBST (0.01M Tris-NaCl, pH 7.6 buffer), rabbit anti-human TDP-43 antibody (Proteintech, 12892-1-AP) and cyclophilin antibody were added to incubate at room temperature for 1.5 h. After washing 4 times with TBST, goat anti-rabbit IgG (HRP) antibody (Abcam, ab6721) secondary antibody was added to incubate at room temperature for 1 h. After washing 4 times with TBST, the PVDF membrane was placed on a clean imaging plate, Immobilon Western HRP Substrate (MILLIPORE, WBKLS0100) was added for color development, and the membrane was photographed under a biomolecular imager, then performing quantitative analysis by using Image J.

The results show that, the molecular weight of the recombinant TDP-43 protein monomer is about 43 kDa, the molecular weight of the high molecular weight TDP-43 protein (HMW) is >55 kDa, and the molecular weight of the low molecular weight TDP-43 fragment (LMW) is <40 kDa. In addition, in the brain homogenate of ALS model mice, the amounts of TDP-43 monomer, HMW and LMW in the plasminogen group are significantly lower than those in the vehicle control group, and the difference is extremely significant (*** represents P < 0.001, ** represents P < 0.01) (Fig. 3). This suggests that plasminogen can promote the cleavage of TDP-43 in the brain homogenate of ALS model mice.

### Example 3: Plasminogen Promotes TDP-43 Degradation in Spinal Cord Tissue of Amyotrophic Lateral Sclerosis Model Mice

Six SOD1-G93A mice aged 10-15 weeks were randomly divided into two groups: 3 mice in vehicle control group, and 3 mice in plasminogen group. The vehicle control group mice were injected with the vehicle at 5 ml/kg by tail vein, and the plasminogen group mice were injected with plasminogen (10 mg/ml) at 50 mg/kg body weight by tail vein. The mice were killed 24 hours after administration, and the spinal cords were obtained. After homogenization, TDP-43 was detected by western blot.

The results show that, the amounts of TDP-43 monomer and low molecular weight TDP-43 in the spinal cord tissue of mice in the plasminogen group are significantly lower than those in the vehicle group, and the statistical difference is significant (* represents P<0.05) (Fig. 4). This indicates that plasminogen can promote the degradation of TDP-43 in the spinal cord tissue of ALS model mice.

### Example 4: Plasminogen Promotes TDP-43 Degradation in Brain Tissue of Amyotrophic Lateral Sclerosis Model Mice

Nine C57BL/6J female mice aged 6-7 weeks were selected and weighed before modeling. After weighing, all mice were randomly divided into two groups: 3 mice in blank control group, and 6 mice in model group. After grouping, mice in the sham operation group and model group were anesthetized with intraperitoneal injection of tribromoethanol at a dose of 20 mL/kg. Model mice were located in the hippocampus according to the mouse stereotaxic atlas (based on the coordinates of the bregma: AP-2.54 mm, ML±2 mm, DV-2.4 mm), and each mouse was slowly microinjected bilaterally. Mice in the sham operation group were only drilled at the coordinate location point without injection [4]. The mice in the model group were injected with TDP-43 solution at an injection rate of 0.5 µL/min and an injection volume of 3 µL/side. After the injection, the syringe stayed for 5 minutes and then slowly withdrew. Three days after the brain localization injection, all mice were weighed, and the mice in the model group were intraperitoneally injected with 5 mg/kg LPS solution according to their body weight. Then the mice in the model group were randomly divided into two groups: 3 mice in the plasminogen group and 3 mice in the vehicle group. 24 hours after LPS injection, the mice in the sham operation group and the vehicle group were injected with 5 mL/kg of the vehicle by tail vein of, and the mice in the plasminogen group were injected with 50 mg/kg of plasminogen by tail vein. The vehicle and plasminogen were administered continuously for 3 days. Two hours after the third administration, the mice were killed, and their brain tissues were obtained. After homogenization, TDP-43 was detected by western blot.

The results show that, the amounts of TDP-43 monomer and low molecular weight TDP-43 in the brain tissue of mice in the plasminogen group are significantly lower than those in the vehicle group, and the statistical difference is significant (* represents P<0.05) (Fig. 5). This indicates that plasminogen can promote the degradation of TDP-43 in the brain tissue of ALS model mice.

### Example 5: Plasminogen is Enriched in Spinal Cord Tissue of Amyotrophic Lateral Sclerosis Model Mice and Co-localized with TDP-43 in Cells

Five wild-type male mice and nine male SOD1-G93A mice of similar week-age were selected. Wild-type mice served as the blank control group, and SOD1-G93A mice were observed and recorded from the time of hind leg tremor when they became ill at week 14, and the onset time of each mouse was recorded. Administering to mice 14 days after the onset of the disease. All mice were randomly divided into a vehicle group and a plasminogen group according to the onset of the disease: 5 mice in the vehicle group, which were injected with 0.1 ml of vehicle (sodium citrate buffer)/mouse by tail vein every day; 4 mice in the plasminogen group, which were injected with 1 mg of plasminogen/0.1 ml of vehicle /mouse by tail vein every day. The vehicle and plasminogen were administered continuously under SPF environment, and the samples were collected at the near-death stage. The longest administration was 61 days. The spinal cord tissue collected was fixed in formalin fixative solution. The fixed tissues were dehydrated with gradient alcohol and transparentized with xylene before being embedded in paraffin. The thickness of tissue sections was 3 µm, and the sections were dewaxed and rehydrated and then washed once. The sections were immersed in antigen retrieval working solution (0.01 M sodium citrate buffer) to perform microwave retrieval: preheating for 5 minutes, high heating for 2 minutes, and low heating for 15 minutes. The tissue was circled with a PAP pen, then incubating in 3% hydrogen peroxide for 15 minutes, and washing twice with 0.01 M PBS for 5 minutes each time, blocking with 5% normal goat serum (Vector laboratories, Inc., USA) for 30 minutes. After the time for blocking is up, the goat serum is discarded, adding anti-plasminogen antibody (self-produced), then incubating at 4°C overnight, and washing twice with 0.01M PBS for 5 minutes each time. The goat anti-rabbit IgG (HRP) antibody (Abcam) secondary antibody was added to incubate at room temperature for 1 hour, then washing twice with 0.01M PBS for 5 minutes each time. The green fluorescence color development of the corresponding anti-plasminogen secondary antibody was performed according to the instructions of the XTSA520 IHC kit (Alpha X Biotech, AXT6202500), washing 3 times with PBS for 5 minutes each time. The above antigen retrieval and blocking procedures were repeated, and then anti-TDP-43 antibody (Proteintech, 12892-1-AP) staining was performed, then incubating at 37°C for 1 hour, washing 3 times with PBS for 5 minutes each time. The goat anti-rabbit IgG (HRP) antibody (Abcam) secondary antibody was added to incubate at room temperature for 1 hour, then washing twice with 0.01M PBS for 5 minutes each time. The goat anti-rabbit IgG (HRP) antibody (Abcam) secondary antibody was added to incubate at room temperature for 1 hour, then washing twice with 0.01M PBS for 5 minutes each time. The red fluorescence color development of the corresponding anti-TDP-43 secondary antibody was performed according to the instructions of the XTSA620 IHC kit (Alpha X Biotech, AXT6502500), washing 3 times with PBS for 5 minutes each time. Nuclear staining with DAPI (BOSTER, 11K16B77) was performed, washing 3 times with PBS for 5 minutes each time. Dehydration was performed with gradient alcohol, then transparentizing with xylene and sealing with neutral gum; the sections were observed and photographed under a 400x optical microscope.

The results show that, the positive staining of plasminogen (green fluorescence) in the spinal cord tissue of the plasminogen group is significantly more than that of the vehicle group, indicating that the administered plasminogen can enter the spinal cord tissue and be enriched in the spinal cord tissue. In addition, plasminogen is present in the cytoplasm (as indicated by △) and in the nucleus (as indicated by 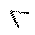 ). Plasminogen co-localizes with TDP-43 (red fluorescence) in the cytoplasm (as indicated by ▲) and in the nucleus (as indicated by 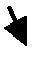 ). The level of TDP-43 in the spinal cord tissue of the plasminogen group was lower than that in the vehicle group, and the co-localization of plasminogen and TDP-43 in the plasminogen group was more than that in the vehicle group (Fig. 6). This indicates that in ALS model mice, plasminogen can enter the spinal cord tissue, enter the cells, co-localize with TDP-43, and degrade TDP-43.

### Example 6: Plasminogen is Enriched in Muscle Tissue of Amyotrophic Lateral Sclerosis Model Mice and Co-localized with TDP-43 in Cells

Five wild-type male mice and nine male SOD1-G93A mice of similar week-age were selected. Wild-type mice served as the blank control group, and SOD1-G93A mice were observed and recorded from the time of hind leg tremor when they became ill at week 14, and the onset time of each mouse was recorded. Administering to mice 14 days after the onset of the disease. All mice were randomly divided into a vehicle group and a plasminogen administration group according to the onset of the disease: 5 mice in the vehicle group, which were injected with 0.1 ml of vehicle (sodium citrate buffer)/mouse by tail vein every day; 4 mice in the plasminogen administration group, which were injected with 1 mg of plasminogen/0.1 ml of vehicle/mouse by tail vein every day. The vehicle and plasminogen were administered continuously under SPF environment, and the samples were collected at the near-death stage. The longest administration was 61 days. The gluteal muscle tissue collected was fixed in formalin fixative solution. The fixed tissues were dehydrated with gradient alcohol and transparentized with xylene before being embedded in paraffin. The thickness of tissue sections was 3 µm, and the sections were dewaxed and rehydrated and then washed once. The sections were immersed in antigen retrieval working solution (0.01 M sodium citrate buffer) to perform microwave retrieval: preheating for 5 minutes, high heating for 2 minutes, and low heating for 15 minutes. The tissue was circled with a PAP pen, then incubating in 3% hydrogen peroxide for 15 minutes, and washing twice with 0.01 M PBS for 5 minutes each time, blocking with 5% normal goat serum (Vector laboratories, Inc., USA) for 30 minutes. After the time for blocking is up, the goat serum is discarded, adding anti-plasminogen antibody (self-produced), then incubating at 4°C overnight, and washing twice with 0.01M PBS for 5 minutes each time. The goat anti-rabbit IgG (HRP) antibody (Abcam) secondary antibody was added to incubate at room temperature for 1 hour, then washing twice with 0.01M PBS for 5 minutes each time. The green fluorescence color development of the corresponding anti-plasminogen secondary antibody was performed according to the instructions of the XTSA520 IHC kit (Alpha X Biotech, AXT6202500), washing 3 times with PBS for 5 minutes each time. The above antigen retrieval and blocking procedures were repeated, and then anti-TDP-43 antibody (Proteintech, 12892-1-AP) staining was performed, then incubating at 37°C for 1 hour, washing 3 times with PBS for 5 minutes each time. The goat anti-rabbit IgG (HRP) antibody (Abcam) secondary antibody was incubated with at room temperature for 1 hour, and then washed twice with 0.01M PBS for 5 minutes each time. The goat anti-rabbit IgG (HRP) antibody (Abcam) secondary antibody was added to incubate at room temperature for 1 hour, then washing twice with 0.01M PBS for 5 minutes each time. The red fluorescence color development of the corresponding anti-TDP-43 secondary antibody was performed according to the instructions of the XTSA620 IHC kit (Alpha X Biotech, AXT6502500), washing 3 times with PBS for 5 minutes each time. Nuclear staining with DAPI (BOSTER, 11K16B77) was performed, then washing 3 times with PBS for 5 minutes each time. Dehydration was performed with gradient alcohol, then transparentizing with xylene and sealing with neutral gum; the sections were observed and photographed under a 400x optical microscope.

The results show that, the positive staining of plasminogen (green fluorescence) in the muscle tissue of the plasminogen group is significantly more than that of the vehicle group, indicating that plasminogen can be enriched in the muscle tissue. In addition, plasminogen is present in the cytoplasm (as indicated by △) and in the nucleus (as indicated by 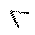 ). Plasminogen co-localizes with TDP-43 (red fluorescence) in the cytoplasm (as indicated by **▲**) and in the nucleus (as indicated by 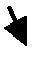 ) (Fig. 7). This indicates that in ALS model mice, plasminogen can be enriched in muscle tissue, enter cells, and co-localize with TDP-43.

### Example 7: Plasminogen Promotes TDP-43 Degradation in Brain Tissue of Okadaic Acid-Induced Dementia Model Mice

Ten B6SJL-Tg (APPSwFlLon, PSEN1*M146L*L286V) 6799Vas/Mmjax (hereinafter referred to as FAD) female mice aged 30-32 weeks (purchased from Jackson lab, stock number: 034840) were taken to weigh before modeling as the model group, and five C57 female mice aged 6-7 weeks were selected as the blank group. After grouping, mice in the blank group and model group were anesthetized by intraperitoneal injection of tribromoethanol at a dose of 20 mL/kg. Model mice were located in the basolateral amygdala according to the mouse stereotaxic atlas (according to the coordinates of the bregma: AP-1.94 mm, NIL±3.15 mm, DV-4.5 mm), and each mouse was slowly microinjected bilaterally. Mice in the blank group were only drilled at the coordinate location point without injection [5]. The mice in the model group were injected with 50ng/µL okadaic acid (manufacturer: Shanghai Yuanye Biotechnology Co., Ltd., catalog number S30686-25ug:) solution, the injection rate was 0.5µL/min, the injection volume was 2µL, and after the injection, the syringe stayed for 6 minutes and then slowly withdrew. Three days after the brain localization injection, all mice were weighed, and the mice in the model group were randomly divided into two groups according to their body weight: 5 mice in the plasminogen group, and 5 mice in the vehicle group. The mice in the blank group and the vehicle group were injected with 5 mL/kg of the vehicle by tail vein, and the mice in the plasminogen group were injected with 50 mg/kg of plasminogen by tail vein. The mice were killed 6 hours after the single administration and the brain tissues were obtained. After homogenization, TDP-43 was detected by western blot.

The results show that, the levels of TDP-43 monomer and low molecular weight TDP-43 in the brain tissue of mice in the plasminogen group are significantly lower than those in the vehicle group (Fig. 8). This indicates that plasminogen can promote the degradation of TDP-43 in the brain tissue of okadaic acid-induced dementia model mice.

### Example 8: Plasminogen Promotes TDP-43 Degradation in Nuclei of Renal Cells in Amyotrophic Lateral Sclerosis Model Mice

SOD1-G93A mice (Jackson Laboratory, Stock Number: 004435) and C57BL/6J mice aged 9-10 weeks were selected. SOD1-G93A mice were randomly divided into two groups, vehicle group and plasminogen group, and C57BL/6J mice were used as normal control group, with 3 mice in each group. The mice in the vehicle group were injected with 5 mL/kg vehicle (10 mM citric acid sodium citrate solution, pH 7.4) via the tail vein every day, and the mice in the plasminogen group were injected with 50 mg/kg plasminogen via the tail vein every day. No treatment was administered to the normal control mice. The mice were killed after 7 days, and kidney tissues were obtained. The sampled kidney tissue was placed in pre-cooled RPMI-1640 (Sigma-Aldrich) culture medium on ice. After rinsing with PBS, the kidney tissue was cut into small pieces and then incubated with 0.25% trypsin for digestion at 37°C for 10 min, shaking every 2 min. Digestion was terminated by adding DMEM medium containing 10% fetal newborn calf serum. After centrifugation (1500 rpm, 5 minutes), the supernatant was removed to obtain a single cell pellet. 200 µL of plasma protein extraction reagent (the volume of a pellet of 2×10⁶ cells is about 20 µL or 40 mg) (Solarbio, R0050) was added for every 20 µL of cell pellet. The cell pellet was completely dispersed into a single-cell suspension by pipette blow or high-speed vortex for 15 seconds, then ice-bathing for 10 minutes, vortexing vigorously at maximum speed for 10 seconds, and centrifuging at 12,000-16,000 g for 10 minutes at 4°C. The supernatant is the extracted cytoplasmic protein, and the supernatant is immediately sucked up into a pre-cooled sample tube for later use. The precipitate is the cell nucleus. The remaining supernatant was completely aspirated (to avoid contamination by cytoplasmic proteins), then adding 50-100 µL of nuclear protein extraction reagent. The precipitate was completely dispersed by pipette blow or high-speed vortex for 15 seconds (can be extended appropriately), then ice-bathing for 10 minutes, vortexing vigorously at maximum speed for 10 seconds and centrifuging at 12,000-16,000 g for 10 minutes at 4°C. The supernatant was immediately sucked up by pipette into a pre-cooled sample tube; this is the extracted nuclear protein. The extracted nuclear protein was tested by western blot for TDP-43.

The results show that, the level of TDP-43 in the renal cell nuclei of the plasminogen group is significantly lower than that of the vehicle group (Figs. 9A-B). This suggests that plasminogen can promote the degradation of TDP-43 in the nucleus of renal cells.

### Example 9: Plasminogen Promotes TDP-43 Degradation in Cytoplasm and Nucleus of NSC34 Cells Treated with Okadaic Acid

10⁶ NSC34 cells (Otwo Biotech, HTX1846) were inoculated in a 9 cm² culture dish, and cultured in DMEM medium (Gibco, 11965092) containing 10% fetal bovine serum (EVERY GREEN, 11011-8611). The cells were placed in a carbon dioxide incubator for culture at 37.0°C and 5% CO₂. After the cells grew for 48 hours and reached about 80%-90% abundance, the medium was changed, and subsequent experiments were performed. The cells were divided into 4 groups: blank control group, vehicle group, plasminogen group and plasminogen+EACA group. The cells in the blank control group were not treated after the medium was changed; the cells in the vehicle group, plasminogen group and plasminogen+EACA group were exposed to okadaic acid (OA) (Shanghai yuanye Bio-Technology, S30686-25ug) at a concentration of 2.5ng/µL. After 24 hours of okadaic acid stimulation, the vehicle was added to the cell culture medium of the vehicle group, plasminogen (0.5 mg/mL) was added to the cell culture medium of the plasminogen group, and plasminogen (final concentration of 0.5 mg/mL) and aminocaproic acid (EACA) (20 mM) were added to the cell culture medium of the plasminogen+EACA group. After adding plasminogen for 24 hours, the cells were harvested. The culture supernatant was sucked off, washing with 1×PBS, digesting with 1mL of 0.25% pancreatic enzyme for 2-3 minutes, then stopping digestion with 5-6mL of DMEM complete medium when the cells were obviously detached. The cells were slowly blown, and the suspension was collected into a centrifuge tube, centrifuging at 1500rpm for 5min to remove the supernatant, resuspending with pre-cooled 1×PBS, and counting the cells. 200 µL of plasma protein extraction reagent (the volume of a pellet of 2×10⁶ cells is about 20 µL or 40 mg) (Solarbio, R0050) was added for every 20 µL of cell pellet. The cell pellet was completely dispersed into a single-cell suspension by pipette blow or high-speed vortex for 15 seconds, then ice-bathing for 10 minutes, vortexing vigorously at maximum speed for 10 seconds and centrifuging at 12,000-16,000 g for 10 minutes at 4°C. The supernatant is the extracted cytoplasmic protein, and the supernatant is immediately sucked up into a pre-cooled sample tube for later use. The precipitate is the cell nucleus, and the remaining supernatant was completely aspirated (to avoid contamination by cytoplasmic proteins), then adding 50-100 µL of nuclear protein extraction reagent. The precipitate is completely dispersed by pipette blow or high-speed vortex for 15 seconds (can be extended appropriately), then ice-bathing for 10 minutes, vortexing vigorously at maximum speed for 10 seconds and centrifuging at 12,000-16,000 g for 10 minutes at 4°C. The supernatant was immediately sucked up by pipette into a pre-cooled sample tube; this is the extracted nuclear protein. The extracted nuclear protein was tested by western blot for TDP-43.

Aminocaproic acid (EACA) is a lysine analog that blocks the high-affinity lysine binding site on plasminogen [6].

The results show that, the levels of TDP-43 in the cytoplasm and nucleus of the plasminogen group are significantly lower than those in the nucleus of the vehicle group, and the addition of EACA can completely inhibit the effect of plasminogen on TDP-43 (Fig. 10A-D). These results suggest that plasminogen can degrade TDP-43 in the cytoplasm and nucleus, and this effect of plasminogen is closely related to the lysine binding site in its structure.

### Example 10: Plasminogen Promotes Increase of Plasminogen Level and Plasmin Activity Level in Cytoplasm and Nucleus of NSC34 Cells Treated with Okadaic Acid

10⁶ NSC34 cells (Otwo Biotech, HTX1846) were inoculated in a 9 cm² culture dish and cultured in DMEM medium (Gibco, 11965092) containing 10% fetal bovine serum (EVERY GREEN, 11011-8611). The cells were placed in a carbon dioxide incubator for culture at 37.0°C and 5% CO₂. After the cells grew for 48 hours and reached about 80%-90% abundance, the medium was changed and subsequent experiments were performed. The cells were divided into three groups: vehicle group, plasminogen group, and plasminogen+EACA group. The cells in the vehicle group, plasminogen group and plasminogen+EACA group were exposed to okadaic acid (OA) (Shanghai Yuanye Bio-Technology, S30686-25ug) at a concentration of 2.5 ng/µL. After 24 hours of okadaic acid stimulation, the vehicle was added to the cell culture medium of the vehicle group, plasminogen (0.5 mg/mL) was added to the cell culture medium of the plasminogen group, and plasminogen (final concentration of 0.5 mg/mL) and aminocaproic acid (EACA) (20 mM) were added to the cell culture medium of the plasminogen+EACA group. After adding plasminogen for 24 hours, the cells were harvested. The culture supernatant was sucked off, washing with 1× PBS, digesting with 1 mL of 0.25% trypsin for 2-3 minutes, stopping digestion with 5-6 mL of DMEM complete medium when the cells were obviously detached. The cells were slowly blown, then the suspension was collected into a centrifuge tube, centrifuging at 1500 rpm for 5 min to remove the supernatant, resuspending in pre-cooled 1× PBS and counting the cells. 200 µL of plasma protein extraction reagent (the volume of a pellet of 2×10⁶ cells is approximately 20 µL or 40 mg) (Solarbio, R0050) was added for every 20 µL of cell pellet. The cell pellet was completely dispersed into a single-cell suspension by pipette blow or high-speed vortex for 15 seconds, ice-bathing for 10 minutes, vortexing vigorously at maximum speed for 10 seconds and centrifuging at 12,000-16,000 g for 10 minutes at 4°C. The supernatant is the extracted cytoplasmic protein, and the supernatant is immediately sucked up into a pre-cooled sample tube for later use. The precipitate is the cell nucleus, and the remaining supernatant was completely aspirated (to avoid contamination by cytoplasmic proteins), then adding 50-100 µL of nuclear protein extraction reagent. The precipitate is completely dispersed by pipette blow or high-speed vortex for 15 seconds (can be extended appropriately), then ice-bathing for 10 minutes, vortexing vigorously at maximum speed for 10 seconds and centrifuging at 12,000-16,000 g for 10 minutes at 4°C. The supernatant was immediately sucked up by pipette into a pre-cooled sample tube; this is the extracted nuclear protein.

After cell lysis, detection was performed according to the instructions of Human Plasminogen ELISA Kit (manufacturer: AssayMax, catalog number: EP1200-1). The human plasminogen working standard in the kit was used as the internal standard to calibrate the concentration of each sample. The calibrated concentration was divided by the total protein concentration to calculate the amount of plasminogen per unit of total protein in each sample and perform statistical analysis.

The plasmin activity was detected by enzyme substrate kinetic method. 85 µL/well of standard solutions at seven different concentrations, blank, and samples were added to the ELISA plate in sequence, and then 15 µL of 20 mM S-2251 solution (Chromogenix, 82033239) was added to each well to incubate at 37°C. Starting from 0 min of reaction, the A405 absorbance value was read in a multifunctional microplate reader every 5 min until the reaction was 90 min. All reactions were fitted with a straight line by time and absorbance, and the slope of the straight line was the reaction rate of the standard/sample (ΔA405/min). Finally, the potency value of the standard substance and △A405/min were used as a standard curve to calculate the potency of the measured samples.

The results show that, the levels of human plasminogen and plasmin activity in the cytoplasm and nucleus of the plasminogen group are significantly higher than those in the vehicle group, and the statistical differences are significant; the addition of EACA can completely inhibit these effects of plasminogen (Figs. 11A-D). This suggests that plasminogen can enter cells and even the cell nucleus, increasing plasmin activity, and that the entry of plasminogen into cells and cell nuclei is closely related to its lysine binding activity.

### Example 11: Administration of Plasminogen Promotes Increase of Blood Plasminogen Level in SOD1-G93A Mice

Twenty-seven 10-15 week-age B6.Cg-Tg (SOD1-G93A) 1Gur/J(SOD1-G93A) mice (pedigree number: 004435) (referred to as SOD1-G93A mice) (breeding mice were purchased from Jackson Laboratory, USA) were randomly divided into three groups: three in the vehicle control group, 12 in the 6 mg/kg plasminogen group, and 12 in the 50 mg/kg plasminogen group. The mice in the vehicle control group were injected with vehicle at 5 ml/kg by tail vein, the mice in the 6 mg/kg plasminogen group were injected with plasminogen (1.2 mg/ml) at 6 mg/kg body weight by tail vein, and the mice in the 50 mg/kg plasminogen group were injected with plasminogen (10 mg/ml) at 6 mg/kg body weight by tail vein. The mice in the vehicle control group were killed 2 hours after administration, and their blood was collected. Three mice in the 6 mg/kg plasminogen group and three mice in the 50 mg/kg plasminogen group were killed at 2, 6, 12 and 24 hours after administration, and their blood was collected. After the blood was centrifuged (3500 rpm, 10 min, 4°C), the supernatant was taken to test according to the instructions of Human Plasminogen ELISA Kit (manufacturer: AssayMax, catalog number: EP1200-1). The concentration of each sample was calibrated by using human plasminogen working standard as the internal standard. The calibrated concentration was divided by the total protein concentration to calculate the amount of plasminogen per unit of total protein in each sample and perform statistical analysis.

The ELISA results of plasma level in SOD1-G93A mice show that, the plasma plasminogen levels of SOD1-G93A mice increase significantly after tail vein injection of 50 mg/kg and 6 mg/kg plasminogen, and the plasminogen level in the 50 mg/kg group is significantly higher than that in the 6 mg/kg group. Plasminogen level gradually decrease 2 hours after administration, and the plasminogen is almost completely metabolized within 12-24 hours (Fig. 12).

The results show that: (1) the plasminogen level in plasma has a dose-dependent effect, and the higher the concentration of plasminogen administered, the higher the plasma plasminogen level; (2) the plasminogen level in plasma has a time-dependent effect, first increasing and then gradually decreasing from 2 to 12 hours.

### Example 12: Administration of Plasminogen Promotes Increase of Plasminogen Level in Brain Tissue of SOD1-G93A Mice

Brain tissue was obtained from the mice sacrificed in Example 11, homogenizing and detecting according to the instructions of Human Plasminogen ELISA Kit (manufacturer: AssayMax, catalog number: EP1200-1). The human plasminogen working standard in the kit was used as the internal standard to calibrate the concentration of each sample. The calibrated concentration was divided by the total protein concentration to calculate the amount of plasminogen per unit of total protein in each sample and perform statistical analysis.

The ELISA results of plasminogen level in the brain of SOD1-G93A mice show that, the plasminogen levels in the brain tissue of SOD1-G93A mice increase significantly after tail vein injection of 50 mg/kg and 6 mg/kg plasminogen, and the plasminogen level in the 50 mg/kg plasminogen group is significantly higher than that in the 6 mg/kg plasminogen group. Plasminogen level gradually decreases 2 hours after administration, and the plasminogen is almost completely metabolized within 12-24 hours (Fig. 13A). 2, 6, and 12 hours after administration of plasminogen, the ratios of brain tissue plasminogen level to blood plasminogen level are 3.47%, 4.94%, and 6.79%, respectively (Fig. 13B).

The results show that: (1) under physiological and pathological conditions, administration of plasminogen can promote plasminogen to cross the blood-brain barrier and accumulate in brain tissue; (2) intravenous injection of plasminogen into mice significantly increases the plasminogen level in brain tissue; (3) the accumulation of plasminogen in brain tissue has a time-dependent effect, first increasing, then gradually decreasing from 2 to 12 hours, and the plasminogen is almost completely metabolized within 12-24 hours; (4) the accumulation of plasminogen in brain tissue has a dose-dependent effect, and the higher the dose administered, the higher the plasminogen level in brain tissue.

### Example 13: Administration of Plasminogen Promotes Increase of Plasminogen Level in Spinal Cord Tissue of SOD1-G93A Mice

Spinal cord tissue was obtained from the mice sacrificed in Example 11, homogenizing and detecting according to the instructions of Human Plasminogen ELISA Kit (manufacturer: AssayMax, catalog number: EP1200-1). The human plasminogen working standard in the kit was used as the internal standard to calibrate the concentration of each sample. The calibrated concentration was divided by the total protein concentration to calculate the amount of plasminogen per unit of total protein in each sample and perform statistical analysis.

The ELISA results of plasminogen level in the spinal cord of SOD1-G93A mice show that the levels of plasminogen in the spinal cord of SOD1-G93A mice increase significantly after tail vein injection of 50 mg/kg and 6 mg/kg plasminogen, and the plasminogen level in the 50 mg/kg group is significantly higher than that in the 6 mg/kg group. Plasminogen level gradually decreases 2 hours after administration, and the plasminogen is almost completely metabolized within 12-24 hours (Fig. 14).

The results show that: (1) plasminogen administered at physiological dose level can cross the blood-brain barrier of SOD1-G93A mice and accumulate in the spinal cord tissue; (2) the accumulation of plasminogen in the spinal cord is dose-dependent, and the higher the dose of plasminogen administered, the greater the accumulation; and (3) the accumulation of plasminogen in the spinal cord is time-dependent, increasing first, then gradually decreasing from 2 to 12 hours, and the plasminogen is almost completely metabolized within 12-24 hours.

### Example 14: Administration of Plasminogen Promotes Increase of Plasminogen Level and Plasmin Activity Level in Brain Tissue of SOD1-G93A Mice

Eight 22-week-age SOD1-G93A mice were randomly divided into two groups: a vehicle group and a plasminogen group, with 4 mice in each group. 2.5 mg/mL bacterial lipopolysaccharide (LPS) (Beijing Solebow Technology Co., Ltd., L8880) was administered to the mice in the vehicle group and the plasminogen group via intratracheal instillation, with a modeling dose of 5 mg/kg. Four C57 mice of the same age were selected as normal control mice. Administration began 3 days after LPS treatment. The mice in the blank group and the vehicle group were injected with 5 mL/kg of the vehicle by tail vein, and the mice in the plasminogen group were injected with 50 mg/kg of plasminogen by tail vein. The mice were killed 6 hours after the single administration, and the brain tissues were obtained. After homogenization, the plasminogen level was detected by ELISA, and the plasmin activity was detected by enzyme substrate method.

The results show that, the plasminogen level and plasmin activity in the brain tissue homogenate of the mice in the plasminogen group is significantly higher than that in the vehicle group, and the statistical difference is significant (Figs. 15A-D). These results suggest that intravenous administration of plasminogen can promote increase of plasminogen level and plasmin activity in brain tissue.

### Example 15: Administration of Plasminogen Promotes Increase of Plasminogen Level in Nuclei of Brain Tissue, Spinal Cord Tissue and Kidney Tissue of SOD1-G93A Mice

Ten 9-week-age SOD1-G93A mice were randomly divided into two groups: a vehicle group and a plasminogen group, with 5 mice in each group. Five C57 mice of the same week-age were selected as normal control mice. The mice in the normal group and the vehicle group were injected with 5 mL/kg of the vehicle by tail vein, and the mice in the plasminogen group were injected with 50 mg/kg of plasminogen by tail vein every day. The vehicle and plasminogen were administered continuously for 7 days. The mice were killed 7 days later, and brain, spinal cord and kidney tissues were collected. The tissue was cut into small pieces to digest with 0.25% trypsin (Beyotime Biotechnology, C0201-500 mL), then filtering with a 200-mesh cell sieve to obtain a single cell suspension. 200 µL of plasma protein extraction reagent (the volume of a pellet of 2×10⁶ cells is approximately 20 µL or 40 mg) (Solarbio, R0050) was added for every 20 µL of cell pellet. The cell pellet was completely dispersed by pipette blow or high-speed vortex for 15 seconds into a single-cell suspension, ice-bathing for 10 minutes, vortexing vigorously at maximum speed for 10 seconds and centrifuging at 12,000-16,000 g for 10 minutes at 4°C. The supernatant is the extracted cytoplasmic protein, and the supernatant is immediately sucked up into a pre-cooled sample tube for later use. The precipitate is the cell nucleus, and the remaining supernatant was completely aspirated (to avoid contamination by cytoplasmic proteins), then adding 50-100 µL of nuclear protein extraction reagent. The precipitate was completely dispersed by pipette blow or high-speed vortex for 15 seconds (can be extended appropriately), then ice-bathing for 10 minutes, vortexing vigorously at maximum speed for 10 seconds and centrifuging at 12,000-16,000 g for 10 minutes at 4°C. The supernatant was immediately sucked up by pipette into a pre-cooled sample tube; this is the extracted nuclear protein. The extracted nuclear protein was tested for human plasminogen level by ELISA.

The results show that, 7 days after administration of plasminogen, the levels of human plasminogen in the nuclei of brain tissue, spinal cord tissue and kidney tissue of SOD1-G93A mice in the plasminogen group are significantly higher than those in the vehicle group, and the statistical difference is extremely significant (*** represents P<0.001) (Fig. 16). These results suggest that intravenous administration of plasminogen can promote increase of the level of human plasminogen in the nuclei of brain, spinal cord and kidney tissues.

### Example 16: Administration of Plasminogen Promotes Increase of Blood Plasminogen Level in Parkinson's Model Mice

Eighteen 6-week-age male C57BL/6J mice were injected intraperitoneally with 1-methyl-4-phenyl-1,2,3,6-tetrahydropyridine (MPTP) solution at a dose of 35 mg/kg/mouse for 5 consecutive days to establish a Parkinson's model [7]. Preparation of MPTP solution: 45 mg of MPTP (Sigma, M0896) was dissolved in 9 ml of physiological saline solution to prepare a final concentration of 5 mg/ml. The mice were randomly divided into two groups according to their body weight: 6 mice in vehicle group, 12 mice in plasminogen group; and administration was begun to record as day 1. The mice in the plasminogen group were injected with 50 mg/kg body weight of plasminogen solution by tail vein, and the mice in the vehicle group were injected with 5 mL/kg body weight of vehicle solution (10 mM citric acid-sodium citrate solution, pH 7.4) by tail vein. Three mice in the vehicle group were killed respectively 2 and 24 hours after administration, and their blood was collected. Three mice in the plasminogen group were killed 2, 6, 12, and 24 hours after administration, and their blood was collected. After the blood was centrifuged (3500 rpm, 10 min, 4°C), the supernatant was taken to detect according to the instructions of Human Plasminogen ELISA Kit (manufacturer: AssayMax, catalog number: EP1200-1). The concentration of each sample was calibrated by using human plasminogen working standard as the internal standard. The calibrated concentration was divided by the total protein concentration to calculate the amount of plasminogen per unit of total protein in each sample and perform statistical analysis.

The results show that, the plasminogen level in the blood of mice in the plasminogen group is significantly higher than that in the vehicle group. The plasminogen level gradually decreases 2 hours after administration, and the plasminogen is almost completely metabolized within 12-24 hours (Fig. 17) (*** represents P < 0.001).

### Example 17: Administration of Plasminogen Promotes Increase of Plasminogen Level in Brain Tissue of Parkinson's Model Mice

Brain tissue was obtained from the mice sacrificed in Example 16, homogenizing and detecting according to the instructions of Human Plasminogen ELISA Kit (manufacturer: AssayMax, catalog number: EP1200-1). The concentration of each sample was calibrated by using human plasminogen working standard as the internal standard. The calibrated concentration was divided by the total protein concentration to calculate the amount of plasminogen per unit of total protein in each sample and perform statistical analysis.

The results show that, the plasminogen level in the brain tissue of mice in the plasminogen group is significantly higher than that in the vehicle group. The plasminogen level gradually decreases 2 hours after administration, and the plasminogen is almost completely metabolized within 12-24 hours (Fig. 18) (*** represents P < 0.001). The results show that, plasminogen injected by tail vein can cross the blood-brain barrier and promote increase of plasminogen level in the brain tissue of Parkinson's model mice.

### Example 18: Administration of Plasminogen Promotes Increase of Plasminogen Level in Spinal Cord Tissue of Parkinson's Model Mice

Spinal cord tissue was obtained from the mice sacrificed in Example 16, homogenizing and detecting according to the instructions of Human Plasminogen ELISA Kit (manufacturer: AssayMax, catalog number: EP1200-1). The concentration of each sample was calibrated by using human plasminogen working standard as the internal standard. The calibrated concentration was divided by the total protein concentration to calculate the amount of plasminogen per unit of total protein in each sample and perform statistical analysis.

The results show that, the plasminogen level in the spinal cord tissue of the mice in the plasminogen group is significantly higher than that in the vehicle group. The plasminogen level gradually decreases 2 hours after administration, and the plasminogen is almost completely metabolized within 12-24 hours (Fig. 19) (** represents P<0.01, *** represents P<0.001). The results indicate that plasminogen injected by tail vein can cross the blood-brain barrier and promote increase of plasminogen level in the spinal cord tissue of Parkinson's model mice.

### Example 19: Administration of Plasminogen Promotes Increase of Plasminogen Level in Brain and Spinal Cord Tissues of Parkinson's Model Mice

Brain and spinal cord tissues were collected from the mice sacrificed in Example 16, homogenizing and detecting according to the instructions of Human Plasminogen ELISA Kit (manufacturer: AssayMax, catalog number: EP1200-1). The concentration of each sample was calibrated by using human plasminogen working standard as the internal standard. The calibrated concentration was divided by the total protein concentration to calculate the amount of plasminogen per unit of total protein in each sample and perform statistical analysis.

The results show that, the ratios of the plasminogen level in spinal cord tissue to the plasminogen level in blood are 1.24%, 1.16% and 1.46% 2, 6 and 12 hours after administration of plasminogen, respectively; the ratios of the plasminogen level in brain tissue to the plasminogen level in blood are 3.47%, 4.18% and 8.51% 2, 6 and 12 hours after administration of plasminogen, respectively (Fig. 20) (* represents P < 0.05, ** represents P < 0.01). The results show that, plasminogen injected by tail vein can cross the blood-brain barrier and promote increase of plasminogen levels in the brain and spinal cord tissues of Parkinson's model mice.

### Example 20: Plasminogen Promotes Increase of Plasmin Activity in Brain Tissue of Parkinson's Model Mice

Fifteen female mice were taken to weigh before modeling. They were randomly divided into two groups according to their body weight: 5 mice in blank control group, and 10 mice in model group. All mice in the model group were intraperitoneally injected with 5 mg/mL MPTP solution at 35 mg/kg/mouse, and the mice in the blank control group were intraperitoneally injected with 7 mL/kg normal saline. The modeling time was set at 9 am every day, and the injections were continued for 5 days. 24 hours after the last MPTP injection, all mice were weighed and injected intraperitoneally with 5 mg/kg LPS solution. 24 hours after intraperitoneal injection of LPS, the mice in the model group were randomly divided into two groups according to their body weight: 5 mice in the plasminogen group, and 5 mice in the vehicle group. The mice in the vehicle group were injected with the vehicle by tail vein, and the mice in the plasminogen group were injected with 50 mg/kg of plasminogen by tail vein, the mice were given a single dose, and the samples were collected by dissection 2 hours after the administration. The plasmin activity in brain tissue homogenate was detected by enzyme substrate kinetic method. The plasmin activity was detected by enzyme substrate kinetic method. 85 µL/well of seven different concentrations of standard solution, blank, and sample were added to the ELISA plate in sequence, and then 15 µL of 20 mM S-2251 solution (Chromogenix, 82033239) was added to each well to incubate at 37°C. Starting from 0 min of reaction, the A405 absorbance value was read in a multifunctional microplate reader every 5 min until the reaction time was 90 min. All reactions were fitted with a straight line by time and absorbance, and the slope of the straight line was the reaction rate of the standard/sample (ΔA405/min). Finally, the potency value of the standard substance and △A405/min were used to create a standard curve to calculate the potency of the measured samples.

The results show that, the level of plasmin activity in the brain tissue of mice in the plasminogen group is significantly higher than that in the vehicle group, and the statistical difference is significant (Fig. 21) (* represents P<0.05). The results show that, plasminogen injected by tail vein can cross the blood-brain barrier and promote increase of plasmin activity level in the brain tissue of Parkinson's model mice.

### Example 21: Administration of Plasminogen Promotes Increase of Blood Plasminogen Level in Alzheimer's Model Mice

Twenty-seven 16-week-age B6SJL-Tg (APPSwFlLon, PSEN1*M146L* L286V) 6799Vas Mmjax (Stock Number: 034840) (FAD mice for short) (breeding mice were purchased from Jackson Laboratory, USA) were randomly divided into three groups: three mice in vehicle control group, 12 mice in 6 mg/kg plasminogen group, and 12 mice in 50 mg/kg plasminogen group. The mice in the vehicle control group were injected with vehicle at 5 ml/kg by tail vein, the mice in the 6 mg/kg plasminogen group were injected with plasminogen (1.2 mg/ml) at 6 mg/kg body weight by tail vein, and the mice in the 50 mg/kg plasminogen group were injected with plasminogen (10 mg/ml) at 6 mg/kg body weight by tail vein. The mice in the vehicle control group were killed 2 hours after administration, and their blood was collected. Three mice in the 6 mg/kg plasminogen group and three mice in the 50 mg/kg plasminogen group were killed 2, 6, 12 and 24 hours after administration, and their blood was collected. After the blood was centrifuged (3500 rpm, 10 min, 4°C), the supernatant was taken to detect according to the instructions of Human Plasminogen ELISA Kit (manufacturer: AssayMax, catalog number: EP1200-1). The concentration of each sample was calibrated by using human plasminogen working standard as the internal standard. The calibrated concentration was divided by the total protein concentration to calculate the amount of plasminogen per unit of total protein in each sample and perform statistical analysis.

The ELISA results of plasminogen level in plasma of FAD mice show that, the plasma plasminogen levels of FAD mice increase significantly after tail vein injection of 50 mg/kg and 6 mg/kg plasminogen, and the plasminogen level in the 50 mg/kg group is significantly higher than that in the 6 mg/kg group. Plasminogen level gradually decreases 2 hours after administration, and the plasminogen is almost completely metabolized within 12-24 hours (Fig. 22).

The results show that: (1) the plasminogen level in plasma has a dose-dependent effect, and the higher the concentration of plasminogen administered, the more it aggregates; (2) the plasminogen level in plasma has a time-dependent effect, first increasing and then gradually decreasing from 2 to 12 hours.

### Example 22: Administration of Plasminogen Promotes Increase of Plasminogen Level in Brain Tissue of Alzheimer's Model Mice

Brain tissue was obtained from the mice sacrificed in Example 21, homogenizing and detecting according to the instructions of Human Plasminogen ELISA Kit (manufacturer: AssayMax, catalog number: EP1200-1). The concentration of each sample was calibrated by using human plasminogen working standard as the internal standard. The calibrated concentration was divided by the total protein concentration to calculate the amount of plasminogen per unit of total protein in each sample and perform statistical analysis.

The ELISA results of plasminogen level in the brain of FAD mice show that, the plasminogen levels in the brain tissue of FAD mice increase significantly after tail vein injection of 50 mg/kg and 6 mg/kg plasminogen, and the plasminogen level in the 50 mg/kg group is significantly higher than that in the 6 mg/kg group. Plasminogen level gradually decreases 2 hours after administration, and the plasminogen is almost completely metabolized within 12-24 hours (Fig. 23A). The ratios of plasminogen in brain tissue to plasminogen in blood of mice in the 6 mg/kg plasminogen group are 3.59% and 4.23% 2 and 6 hours after plasminogen injection, respectively; the ratios of plasminogen in brain tissue to plasminogen in blood of mice in the 50 mg/kg plasminogen group are 2.49%, 2.31% and 3.32% 2, 6 and 12 hours after plasminogen injection, respectively (Fig. 23B).

The results show that: (1) under physiological and pathological conditions, administration of plasminogen promotes plasminogen to cross the blood-brain barrier and accumulate in brain tissue; (2) intravenous injection of plasminogen significantly increases the plasminogen level in FAD mice; (3) the accumulation of plasminogen in brain tissue is time-dependent, increasing first, then gradually decreasing from 2 to 12 hours, and the plasminogen is almost completely metabolized within 12-24 hours; (4) the accumulation of plasminogen in brain tissue is dose-dependent, and the higher the dose, the higher the plasminogen level in brain tissue.

### Example 23: Administration of Plasminogen Promotes Increase of Plasminogen Level in Brain Tissue of Alzheimer's Model Mice

Brain tissue was obtained from the mice sacrificed in Example 21, and the homogenized tissue was subjected to enzyme substrate kinetic assay for plasmin. 85 µL/well of seven different concentrations of standard solution, blank, and sample were added to the ELISA plate (manufacturer: NUNC, catalog number: 446469) in sequence, and then 15 µL of 20 mM S-2251 solution (manufacturer: Chromogenix, catalog number: 82033239) was added to each well to incubate at 37°C. Starting from 0 min of reaction, the A405 absorbance value was read in a multifunctional microplate reader every 5 min until the reaction time was 90 min. All reactions were fitted with a straight line by time and absorbance, and the slope of the straight line was the reaction rate of the standard/sample (△A405/min). Finally, the potency value of the standard substance and △A405/min were used to create a standard curve to calculate the potency of the measured samples. The plasmin activity per unit of total protein in each sample was calculated.

The results show that, the plasmin activity levels in the brain tissue of FAD mice increase significantly after tail vein injection of 50 mg/kg and 6 mg/kg plasminogen, and the plasminogen level in the 50 mg/kg group is significantly higher than that in the 6 mg/kg group (Fig. 24).

The results show that, the injection of plasminogen into mice significantly increases the level of plasmin in brain tissue. In addition, the plasmin activity in brain tissue has a dose-dependent effect, the higher the dose, the higher the plasminogen level in brain tissue.

### Example 24: Administration of Plasminogen Promotes Increase of Plasminogen Level in Spinal Cord Tissue of Alzheimer's Model Mice

Spinal cord tissue was obtained from the mice sacrificed in Example 21, homogenizing and detecting according to the instructions of Human Plasminogen ELISA Kit (manufacturer: AssayMax, catalog number: EP1200-1). The concentration of each sample was calibrated by using human plasminogen working standard as the internal standard. The calibrated concentration was divided by the total protein concentration to calculate the amount of plasminogen per unit of total protein in each sample and perform statistical analysis.

The ELISA results of plasminogen level in the spinal cord of FAD mice show that, the plasminogen levels in the spinal cord tissue of FAD mice increase significantly after tail vein injection of 50 mg/kg and 6 mg/kg plasminogen, and the plasminogen level in the 50 mg/kg group is significantly higher than that in the 6 mg/kg group. Plasminogen level gradually decreases 2 hours after administration, and the plasminogen is almost completely metabolized within 12-24 hours (Fig. 25A). The ratios of plasminogen in spinal cord tissue to plasminogen in blood of mice in the 6 mg/kg plasminogen group are 0.93% and 1.62% 2 and 6 hours after plasminogen injection, respectively; the ratios of plasminogen in spinal cord tissue to plasminogen in blood of mice in the 50 mg/kg plasminogen group are 0.33%, 0.40% and 1.56% 2, 6 and 12 hours after plasminogen injection, respectively (Fig. 25B).

The results show that: (1) the administration of plasminogen under physiological and pathological conditions can promote plasminogen to cross the blood-brain barrier and accumulate in the spinal cord tissue; (2) intravenous injection of plasminogen into mice significantly increases the plasminogen level in the spinal cord tissue; (3) accumulation of plasminogen in the spinal cord tissue has a time-dependent effect, first increasing, then gradually decreasing from 2 to 12 hours, and the plasminogen is almost completely metabolized within 12-24 hours; (4) accumulation of plasminogen in spinal cord tissue has a dose-dependent effect, and the higher the dose, the higher the plasminogen level in the spinal cord tissue.

### Example 25: Plasminogen Improves Condition of Patients with Amyotrophic Lateral Sclerosis

This trial recruited 9 amyotrophic lateral sclerosis (ALS) patients aged 39-60 years, including 8 patients with limb-type ALS and 1 patient with bulbar-type ALS, one of whom carried a FUS gene mutation. The treatment was approved by the hospital ethics committee. All patients signed informed consent.

Human plasminogen lyophilized powder is dissolved in sterile water at a concentration of 5 mg/ml, and administered to patients via intravenous injection or nebulizer. The basic information of the patients and the use of plasminogen are shown in Table 1.

**Table 1: Basic information and the use of plasminogen for ALS patients**

| Patient ID | Sex | Age (years) | Clinical typing | Situations about administration of plasminogen |
|---|---|---|---|---|
| 1 | Male | 54 | Limb-type | Intravenous injection sometimes accompanied by nebulized inhalation |
| | | | | Intravenous injection: once every 1-2 days, gradually increasing a dosage from 100mg to 300mg each time; |
| | | | | Nebulized inhalation: twice a day, a dosage of 10mg each time, administration for 2 weeks. |
| 2 | Male | 48 | Limb-type | Intravenous injection: once every 2 days, gradually increasing a dosage from 100mg to 200mg each time, administration for 2 weeks. |
| 3 | Male | 39 | Limb-type | Intravenous injection: once every day, gradually increasing a dosage from 50mg to 250mg each time, administration for 12 days. |
| 4 | Female | 44 | Limb-type | Intravenous injection: once every 1-2 days, gradually increasing a dosage from 50mg to 300mg each time, administration for 2 weeks. |
| 5 | Male | 45 | Limb-type | First treatment course, intravenous injection: once every day, gradually increasing a dosage from 50mg to 300mg each time, administration for 11 days. |
| | | | | Second treatment course, nebulized inhalation: twice every 1-3 days, a dosage of 10-60mg each time, administration for 8 weeks, two treatment courses with an interval of 8 weeks. |
| 6 | Male | 48 | Limb-type carrying FUS gene mutation | Intravenous injection: once every 1-2 days, gradually increasing a dosage from 50mg to 400mg each time, administration for 12 days. |
| 7 | Male | 49 | Limb-type | Intravenous injection: once every 1-2 days, gradually increasing a dosage from 50mg to 300mg each time, administration for 2 weeks. |
| 8 | Female | 53 | Bulbar-type | Intravenous injection: once every 1-2 days, gradually increasing a dosage from 50mg to 300mg each time, administration for 2 weeks. |
| 9 | Male | 60 | Limb-type | Nebulized inhalation: twice every day, gradually increasing a dosage from 10mg to 50mg each time, administration for 2 weeks. |

| | | | | |
|---|---|---|---|---|
| Results: plasminogen improves motor function in ALS patients | | | | |

The ALS Functional Rating Scale-Revised (ALSFRS-R, ALS FRS-R) is a widely used and validated assessment tool for monitoring disability progression in ALS patients [8].

The results show that, the ALSFRS-R score of the 9 patients before administration was 20.22±10.01, which increased to 23.13±10.82 after 0.5-4 months of plasminogen administration, an increase of 4.11±5.30 points. In addition, Patient 8 was a patient with bulbar ALS, and her ALSFRS-R score increased rapidly from 20 to 36 points after only 2 weeks of plasminogen administration. Patient 6, who carried a FUS gene mutation, had an increase in his ALSFRS-R score from 27 to 29 after 12 days of plasminogen administration. ALSFRS-R score of Patient 5 dropped from 29 to 25 at 8 weeks after the end of the first treatment course, but in the second treatment course, after receiving only nebulized inhalation of plasminogen for 4 weeks, the score increased to 29. In addition, the maximum number of walking steps of patient 5 increased from more than 40 steps to more than 200 steps in the second treatment course (Figs. 26A-B).

In addition, after administration of plasminogen, the patient's respiratory function, writing ability, speech, swallowing, anxiety and depression, and sleep are significantly improved (Table 2).

The therapeutic effects of plasminogen in ALS were compared with those of two existing FDA-approved drugs for the treatment of ALS, Riluzole and Edaravone. According to literature reports, after 6 months of treatment with Riluzole, the ALSFRS-R score of ALS patients decreased by -7.0±7.1 points; after 6 months of treatment with Edaravone, the ALSFRS-R score of ALS patients decreased by -5.01±0.64 points [9,10]. After 0.5 month of treatment with plasminogen, the ALSFRS-R scores of the 9 ALS patients increased by 4.11±5.30 points, and no adverse events were observed during the administration of plasminogen (Fig. 26C and Table 3).

The above results indicate that plasminogen can safely and effectively treat ALS.

**Table 2: Clinical phenotypes of ALS patients before and after administration of plasminogen**

| Patient ID | | Motor function | Respiratory function | ALSFRS -R scores | Others |
|---|---|---|---|---|---|
| 1 | Before dosing | The head can keep upright for 1 minute, with stiff and swollen arms, contracted fingers on both hands, and trembling fingers. When sitting, the left leg can keep straight for about 41 seconds, the right leg straight for about 18 seconds, and the feet droop and contract inward. | Rapid breathing during speech. | 17 | Slow chewing and swallowing, occasional choking, unclear pronunciation. |
| | After dosing | The head can keep upright for 5-6 minutes, reducing the area of edema in both arms, continuously improving motor function of both hands. When sitting, the legs can keep straight for 80 seconds, reducing foot swelling by 20%. | Extended breathing interval during speech. | 17 | Improved chewing and swallowing abilities, reduced choking frequency, and improved pronunciation. |
| 2 | Before dosing | Stiff and contracted fingers and arms; spasmodic toes, stiff j oints. | Loss of spontaneous breathing ability, using a ventilator, suctioning sputum about 30 times a day. | 0 | Sleeping for 4-5 hours every night, and waking up 4-5 times. |
| | After dosing | The stiffness in both arms has been relieved, and the amplitude and strength of the right shoulder's swing have increased by about 10%. | Suctioning sputum about 5 times a day, with reduced amount of sputum. | 2 | Increasing sleep time by 1-3 hours every night. |
| 3 | Before dosing | The lower limbs have no motor function, from the sole to the knee, with a temperature of 36. 1°C, and the patient cannot move with the feet. | A ventilator is needed for sleeping, and sitting upright is needed for breathing. Blood oxygen saturation is often between 94-96%, occasionally 97%. | 17 | Severe depression, anxiety, waking up 20 times while sleeping at night. |
| | After dosing | The temperature in the plantar area increased to 36.7°C; the motion range of both feet is increased. | Blood oxygen saturation is often between 97-98%. | 17 | Relieved depression and improved sleep quality. |
| 4 | Before dosing | Difficulty writing, for grasping objects, the right hand can hold for 46 seconds, and the left hand for 1-2 seconds. | / | 35 | Deep sleeping for 1.6 hours. |
| | After dosing | Increased writing power, for grasping objects, the right hand can hold for 80 seconds, and the left hand for 8-9 seconds. | / | 36 | Deep sleeping for about 2 hours, good mental state. |
| 5 | Before dosing | When sitting, the head can keep upright for 5 seconds, the right arm cannot be lifted, and objects can be grasped for 1-2 seconds. When sitting, the legs can be straightened to lift 10cm, with a hand gripping force of 6.6kg. | Short breathing, wheezing and coughing during speech; frequently coughing, and occasionally choking. | 23 | Poor sleep quality, easy to wake up; unclear pronunciation and weak speech; the indifferent emotion of reduced interest in the outside world. |
| | After dosing | When sitting, the head can keep upright for 30-60 seconds, the right arm can be lifted 25cm, and objects can be grasped for 1-2 seconds. When sitting, the legs can be straightened to lift 20cm. | Smooth breathing during prolonged speech; reduced frequency of coughing. | 29 | Long lasting and healthy sleep quality; clearer pronunciation, more speaking; emotional improvement, increased number of smiles. |
| 6 | Before dosing | When sitting, the head can keep upright for 30-60 seconds, with muscle stiffness in the back, unable to stand independently. | Rapid breathing during intense activity; oxygen saturation is 95%, rarely reaching 97%. | 27 | Unclear pronunciation, stiff tongue; pale complexion. |
| | After dosing | When sitting, the head can keep upright for 5 minutes, with improvement of back muscle stiffness; improvement of motor function of upper limb; writing flexibility improved by 30%; being able to stand independently, core muscle strength increased after 3 days of treatment. | No shortness of breath during intense activity; the oxygen saturation is usually 97%, occasionally 98%. | 29 | Improved tongue flexibility; a ruddy complexion. |
| 7 | Before dosing | Both hands cannot be lifted, objects can be grasped for 5 seconds, with weak lower limbs, being unable to independently use the toilet, wash, and dress or eat. | / | 20 | Weak and unclear pronunciation, and impatient; subcutaneous tissue relaxation in legs and upper arms. |
| | After dosing | Objects can be grasped for 45 seconds, with improved muscle tone; lower limb strength increased by 20%, and improved walking ability. | / | 24 | Pronunciation improved by 30-40%, reduced severity of impatience. |
| 8 | Before dosing | Painful and stiff neck, weak handwriting, pain in the left arm, stiffness in the right cheek. | When lying flat, there is a feeling of chest tightness and suffocation. | 20 | Difficulty in swallowing and frequent choking; viscous saliva; blurred pronunciation. |
| | After dosing | Reduced neck pain, writing ability returned to normal after 5 days of administration, pain in the left arm reduced by 20%, and lateral facial muscle activity recovered by 50%. | When lying flat, there is no feeling of chest tightness and suffocation. | 36 | Improved swallowing ability, no choking when drinking water, and saliva volume reduced by 50%; slightly clear pronunciation. |
| 9 | Before dosing | The maximum gripping force of the left hand is 16.1kg; the patient can walk for about 5 steps by assistance, but cannot turn over to the right. | The blood oxygen saturation is usually 92-95%, occasionally 96%. | 23 | Significant muscle twitching, depression, inability to speak, choking when drinking water. |
| | After dosing | The maximum gripping force of the left hand is 19.4kg; the patient can walk for about 20 steps by assistance, and can turn over to the right. | The blood oxygen saturation is usually 94-95%, occasionally 97%. | 29 | Muscle beat frequency reduced by 60%, stable emotions, and no depression; increased pronunciation; drinking water without choking. |

| | | | | | |
|---|---|---|---|---|---|
| "/": indicates not observed or recorded | | | | | |

**Table 3: ALSFRS-R scores before and after treatment with plasminogen, Riluzole or Edaravone**

| | Before dosing | Dosing time and mode | After dosing | Scoring change | Side effects |
|---|---|---|---|---|---|
| plasminogen | 20.22 ± 10.01 | 0.5-3 months, mainly intravenous inj ection | 23.13 ± 10.82 | 4.11 ± 5.30 | Not observed |
| Riluzole# | 40.2 ± 4.5 | 6 months, taking orally | 33.2 | -7.0 ± 7.1 | Respiratory failure, liver dysfunction, pleural effusion |
| Edaravone† | 41.9 ± 2.4 | 6 months, intravenous inj ection | 36.89 | - 5.01 ± 0.64 | Bruising, constipation, contact dermatitis, dysphagia, eczema, insomnia, upper respiratory tract inflammation, back pain, etc. |

### REFERENCES

[1] KENNETH C. ROBBINS, LOUIS SUMMARIA, DAVID ELWYN et al. Further Studies on the Purification and Characterization of Human Plasminogen and Plasmin. Journal of Biological Chemistry, 1965, 240 (1) :541-550.
[2] Summaria L, Spitz F, Arzadon L et al. Isolation and characterization of the affinity chromatography forms of human Glu- and Lys-plasminogens and plasmins. J Biol Chem. 1976 Jun 25; 251(12):3693-9.
[3] HAGAN JJ, ABLONDI FB, DE RENZO EC. Purification and biochemical properties of human plasminogen. J Biol Chem. 1960 Apr; 235:1005-10.
[4] Porta S, Xu Y, Restrepo C R , et al. Patient-derived frontotemporal lobar degeneration brain extracts induce formation and spreading of TDP-43 pathology in vivo[J]. Nature Communications, 2018, 9(1).
[5] Kamat P K , Rai S , Nath C . Okadaic acid induced neurotoxicity: An emerging tool to study Alzheimer's disease pathology[J]. Neurotoxicology, 2013, 37:163-172.
[6] Sun Z, Chen YH, Wang P, Zhang J, Gurewich V, Zhang P, Liu JN. The blockage of the high-affinity lysine binding sites of plasminogen by EACA significantly inhibits prourokinase-induced plasminogen activation. Biochim Biophys Acta. 2002 Apr 29;1596(2):182-92.
[7] Ding H, Underwood R, Lavalley N, et al. 14-3-3 inhibition promotes dopaminergic neuron loss and 14-3-30 overexpression promotes recovery in the MPTP mouse model of Parkinson's disease[J]. Neuroscience, 2015, 307:73-82.
[8] Cedarbaum JM, Stambler N, Malta E, Fuller C, Hilt D, Thurmond B, Nakanishi A. The ALSFRS-R: a revised ALS functional rating scale that incorporates assessments of respiratory function. BDNF ALS Study Group (Phase III). J Neurol Sci. 1999 Oct 31; 169 (1-2):13-21.
[9] Shibuya K, Misawa S, Kimura H et al. A single blind randomized controlled clinical trial of mexiletine in amyotrophic lateral sclerosis: Efficacy and safety of sodium channel blocker phase II trial. Amyotroph Lateral Scler Frontotemporal Degener. 2015; 16 (5-6):353-8.
[10] Writing Group; Edaravone (MCI-186) ALS 19 Study Group. Safety and efficacy of edaravone in well defined patients with amyotrophic lateral sclerosis: a randomised, double-blind, placebo-controlled trial. Lancet Neurol. 2017 Jul; 16(7):505-512.

### SEQUENCE LISTINGS

SEQ ID NO:1 (a nucleic sequence of natural plasminogen (Glu-PLG, Glu-plasminogen) without signal peptide): 
gagcctctggatgactatgtgaatacccagggggcttcactgttcagtgtcactaagaagcagctgggagcaggaagtatagaagaatgtgcagcaaaatgtgagga
ggacgaagaattcacctgcagggcattccaatatcacagtaaagagcaacaatgtgtgataatggctgaaaacaggaagtcctccataatcattaggatgagagatgt
agttttatttgaaaagaaagtgtatctctcagagtgcaagactgggaatggaaagaactacagagggacgatgtccaaaacaaaaaatggcatcacctgtcaaaaatg
gagttccacttctccccacagacctagattctcacctgctacacacccctcagagggactggaggagaactactgcaggaatccagacaacgatccgcaggggccct
ggtgctatactactgatccagaaaagagatatgactactgcgacattcttgagtgtgaagaggaatgtatgcattgcagtggagaaaactatgacggcaaaatttccaa
gaccatgtctggactggaatgccaggcctgggactctcagagcccacacgctcatggatacattccttccaaatttccaaacaagaacctgaagaagaattactgtcgt
aaccccgatagggagctgcggccttggtgtttcaccaccgaccccaacaagcgctgggaactttgtgacatcccccgctgcacaacacctccaccatcttctggtccc
acctaccagtgtctgaagggaacaggtgaaaactatcgcgggaatgtggctgttaccgtgtccgggcacacctgtcagcactggagtgcacagacccctcacacac
ataacaggacaccagaaaacttcccctgcaaaaatttggatgaaaactactgccgcaatcctgacggaaaaagggccccatggtgccatacaaccaacagccaagt
gcggtgggagtactgtaagataccgtcctgtgactcctccccagtatccacggaacaattggctcccacagcaccacctgagctaacccctgtggtccaggactgcta
ccatggtgatggacagagctaccgaggcacatcctccaccaccaccacaggaaagaagtgtcagtcttggtcatctatgacaccacaccggcaccagaagacccca
gaaaactacccaaatgctggcctgacaatgaactactgcaggaatccagatgccgataaaggcccctggtgttttaccacagaccccagcgtcaggtgggagtactg
caacctgaaaaaatgctcaggaacagaagcgagtgttgtagcacctccgcctgttgtcctgcttccagatgtagagactccttccgaagaagactgtatgtttgggaat
gggaaaggataccgaggcaagagggcgaccactgttactgggacgccatgccaggactgggctgcccaggagccccatagacacagcattttcactccagagac
aaatccacgggcgggtctggaaaaaaattactgccgtaaccctgatggtgatgtaggtggtccctggtgctacacgacaaatccaagaaaactttacgactactgtgat
gtccctcagtgtgcggccccttcatttgattgtgggaagcctcaagtggagccgaagaaatgtcctggaagggttgtaggggggtgtgtggcccacccacattcctgg
ccctggcaagtcagtcttagaacaaggtttggaatgcacttctgtggaggcaccttgatatccccagagtgggtgttgactgctgcccactgcttggagaagtccccaa
ggccttcatcctacaaggtcatcctgggtgcacaccaagaagtgaatctcgaaccgcatgttcaggaaatagaagtgtctaggctgttcttggagcccacacgaaaag
atattgccttgctaaagctaagcagtcctgccgtcatcactgacaaagtaatcccagcttgtctgccatccccaaattatgtggtcgctgaccggaccgaatgtttcatca
ctggctggggagaaacccaaggtacttttggagctggccttctcaaggaagcccagctccctgtgattgagaataaagtgtgcaatcgctatgagtttctgaatggaag
agtccaatccaccgaactctgtgctgggcatttggccggaggcactgacagttgccagggtgacagtggaggtcctctggtttgcttcgagaaggacaaatacatttta
caaggagtcacttcttggggtcttggctgtgcacgccccaataagcctggtgtctatgttcgtgtttcaaggtttgttacttggattgagggagtgatgagaaataattaa
SEQ ID NO: 2 (a nucleic sequence of natural plasminogen (Glu-PLG, Glu-plasminogen) without signal peptide): 
EPLDDYVNTQGASLFSVTKKQLGAGSIEECAAKCEEDEEFTCRAFQYHSKEQQCVIMAENRKSSIIIRMR
DVVLFEKKVYLSECKTGNGKNYRGTMSKTKNGITCQKWSSTSPHRPRFSPATHPSEGLEENYCRNPDND
PQGPWCYTTDPEKRYDYCDILECEEECMHCSGENYDGKISKTMSGLECQAWDSQSPHAHGYIPSKFPNK
NLKKNYCRNPDRELRPWCFTTDPNKRWELCDIPRCTTPPPSSGPTYQCLKGTGENYRGNVAVTVSGHTC
QHWSAQTPHTHNRTPENFPCKNLDENYCRNPDGKRAPWCHTTNSQVRWEYCKIPSCDSSPVSTEQLAPT
APPELTPVVQDCYHGDGQSYRGTSSTTTTGKKCQSWSSMTPHRHQKTPENYPNAGLTMNYCRNPDADK
GPWCFTTDPSVRWEYCNLKKCSGTEASVVAPPPVVLLPDVETPSEEDCMFGNGKGYRGKRATTVTGTPC
QDWAAQEPHRHSIFTPETNPRAGLEKNYCRNPDGDVGGPWCYTTNPRKLYDYCDVPQCAAPSFDCGKP
 
QVEPKKCPGRVVGGCVAHPHSWPWQVSLRTRFGMHFCGGTLISPEWVLTAAHCLEKSPRPSSYKVILGA
HQEVNLEPHVQEIEVSRLFLEPTRKDIALLKLSSPAVITDKVIPACLPSPNYVVADRTECFITGWGETQGTFG
AGLLKEAQLPVIENKVCNRYEFLNGRVQSTELCAGHLAGGTDSCQGDSGGPLVCFEKDKYILQGVTSWG
LGCARPNKPGVYVRVSRFVTWIEGVMRNN
SEQ ID NO: 3 (a nucleic sequence of natural plasminogen (from swiss prot) with signal peptide): 
atggaacataaggaagtggttcttctacttcttttatttctgaaatcaggtcaaggagagcctctggatgactatgtgaatacccagggggcttcactgttcagtgtcactaa
gaagcagctgggagcaggaagtatagaagaatgtgcagcaaaatgtgaggaggacgaagaattcacctgcagggcattccaatatcacagtaaagagcaacaatg
tgtgataatggctgaaaacaggaagtcctccataatcattaggatgagagatgtagttttatttgaaaagaaagtgtatctctcagagtgcaagactgggaatggaaaga
actacagagggacgatgtccaaaacaaaaaatggcatcacctgtcaaaaatggagttccacttctccccacagacctagattctcacctgctacacacccctcagagg
gactggaggagaactactgcaggaatccagacaacgatccgcaggggccctggtgctatactactgatccagaaaagagatatgactactgcgacattcttgagtgt
gaagaggaatgtatgcattgcagtggagaaaactatgacggcaaaatttccaagaccatgtctggactggaatgccaggcctgggactctcagagcccacacgctca
tggatacattccttccaaatttccaaacaagaacctgaagaagaattactgtcgtaaccccgatagggagctgcggccttggtgtttcaccaccgaccccaacaagcgc
tgggaactttgtgacatcccccgctgcacaacacctccaccatcttctggtcccacctaccagtgtctgaagggaacaggtgaaaactatcgcgggaatgtggctgtta
ccgtgtccgggcacacctgtcagcactggagtgcacagacccctcacacacataacaggacaccagaaaacttcccctgcaaaaatttggatgaaaactactgccgc
aatcctgacggaaaaagggccccatggtgccatacaaccaacagccaagtgcggtgggagtactgtaagataccgtcctgtgactcctccccagtatccacggaac
aattggctcccacagcaccacctgagctaacccctgtggtccaggactgctaccatggtgatggacagagctaccgaggcacatcctccaccaccaccacaggaaa
gaagtgtcagtcttggtcatctatgacaccacaccggcaccagaagaccccagaaaactacccaaatgctggcctgacaatgaactactgcaggaatccagatgccg
ataaaggcccctggtgttttaccacagaccccagcgtcaggtgggagtactgcaacctgaaaaaatgctcaggaacagaagcgagtgttgtagcacctccgcctgtt
gtcctgcttccagatgtagagactccttccgaagaagactgtatgtttgggaatgggaaaggataccgaggcaagagggcgaccactgttactgggacgccatgcca
ggactgggctgcccaggagccccatagacacagcattttcactccagagacaaatccacgggcgggtctggaaaaaaattactgccgtaaccctgatggtgatgtag
gtggtccctggtgctacacgacaaatccaagaaaactttacgactactgtgatgtccctcagtgtgcggccccttcatttgattgtgggaagcctcaagtggagccgaa
gaaatgtcctggaagggttgtaggggggtgtgtggcccacccacattcctggccctggcaagtcagtcttagaacaaggtttggaatgcacttctgtggaggcacctt
gatatccccagagtgggtgttgactgctgcccactgcttggagaagtccccaaggccttcatcctacaaggtcatcctgggtgcacaccaagaagtgaatctcgaacc
gcatgttcaggaaatagaagtgtctaggctgttcttggagcccacacgaaaagatattgccttgctaaagctaagcagtcctgccgtcatcactgacaaagtaatccca
gcttgtctgccatccccaaattatgtggtcgctgaccggaccgaatgtttcatcactggctggggagaaacccaaggtacttttggagctggccttctcaaggaagccc
agctccctgtgattgagaataaagtgtgcaatcgctatgagtttctgaatggaagagtccaatccaccgaactctgtgctgggcatttggccggaggcactgacagttg
ccagggtgacagtggaggtcctctggtttgcttcgagaaggacaaatacattttacaaggagtcacttcttggggtcttggctgtgcacgccccaataagcctggtgtct
atgttcgtgtttcaaggtttgttacttggattgagggagtgatgagaaataattaa
SEQ ID NO: 4 (an amino acid sequence of natural plasminogen (from swiss prot) with signal peptide):
SEQ ID NO: 5 (a nucleic sequence of LYS77-PLG (Lys-plasminogen)): 
aaagtgtatctctcagagtgcaagactgggaatggaaagaactacagagggacgatgtccaaaacaaaaaatggcatcacctgtcaaaaatggagttccacttctccc
cacagacctagattctcacctgctacacacccctcagagggactggaggagaactactgcaggaatccagacaacgatccgcaggggccctggtgctatactactg
atccagaaaagagatatgactactgcgacattcttgagtgtgaagaggaatgtatgcattgcagtggagaaaactatgacggcaaaatttccaagaccatgtctggact
ggaatgccaggcctgggactctcagagcccacacgctcatggatacattccttccaaatttccaaacaagaacctgaagaagaattactgtcgtaaccccgataggga
gctgcggccttggtgtttcaccaccgaccccaacaagcgctgggaactttgtgacatcccccgctgcacaacacctccaccatcttctggtcccacctaccagtgtctg
aagggaacaggtgaaaactatcgcgggaatgtggctgttaccgtgtccgggcacacctgtcagcactggagtgcacagacccctcacacacataacaggacacca
gaaaacttcccctgcaaaaatttggatgaaaactactgccgcaatcctgacggaaaaagggccccatggtgccatacaaccaacagccaagtgcggtgggagtact
gtaagataccgtcctgtgactcctccccagtatccacggaacaattggctcccacagcaccacctgagctaacccctgtggtccaggactgctaccatggtgatggac
agagctaccgaggcacatcctccaccaccaccacaggaaagaagtgtcagtcttggtcatctatgacaccacaccggcaccagaagaccccagaaaactacccaa
atgctggcctgacaatgaactactgcaggaatccagatgccgataaaggcccctggtgttttaccacagaccccagcgtcaggtgggagtactgcaacctgaaaaaa
tgctcaggaacagaagcgagtgttgtagcacctccgcctgttgtcctgcttccagatgtagagactccttccgaagaagactgtatgtttgggaatgggaaaggatacc
gaggcaagagggcgaccactgttactgggacgccatgccaggactgggctgcccaggagccccatagacacagcattttcactccagagacaaatccacgggcg
ggtctggaaaaaaattactgccgtaaccctgatggtgatgtaggtggtccctggtgctacacgacaaatccaagaaaactttacgactactgtgatgtccctcagtgtgc
ggccccttcatttgattgtgggaagcctcaagtggagccgaagaaatgtcctggaagggttgtaggggggtgtgtggcccacccacattcctggccctggcaagtca
gtcttagaacaaggtttggaatgcacttctgtggaggcaccttgatatccccagagtgggtgttgactgctgcccactgcttggagaagtccccaaggccttcatcctac
aaggtcatcctgggtgcacaccaagaagtgaatctcgaaccgcatgttcaggaaatagaagtgtctaggctgttcttggagcccacacgaaaagatattgccttgctaa
agctaagcagtcctgccgtcatcactgacaaagtaatcccagcttgtctgccatccccaaattatgtggtcgctgaccggaccgaatgtttcatcactggctggggaga
aacccaaggtacttttggagctggccttctcaaggaagcccagctccctgtgattgagaataaagtgtgcaatcgctatgagtttctgaatggaagagtccaatccaccg
aactctgtgctgggcatttggccggaggcactgacagttgccagggtgacagtggaggtcctctggtttgcttcgagaaggacaaatacattttacaaggagtcacttct
tggggtcttggctgtgcacgccccaataagcctggtgtctatgttcgtgtttcaaggtttgttacttggattgagggagtgatgagaaataattaa
SEQ ID NO: 6 (an amino acid sequence of LYS77-PLG (Lys-plasminogen)): 
KVYLSECKTGNGKNYRGTMSKTKNGITCQKWSSTSPHRPRFSPATHPSEGLEENYCRNPDNDPQGPWCY
TTDPEKRYDYCDILECEEECMHCSGENYDGKISKTMSGLECQAWDSQSPHAHGYIPSKFPNKNLKKNYC
RNPDRELRPWCFTTDPNKRWELCDIPRCTTPPPSSGPTYQCLKGTGENYRGNVAVTVSGHTCQHWSAQT
PHTHNRTPENFPCKNLDENYCRNPDGKRAPWCHTTNSQVRWEYCKIPSCDSSPVSTEQLAPTAPPELTPV
VQDCYHGDGQSYRGTSSTTTTGKKCQSWSSMTPHRHQKTPENYPNAGLTMNYCRNPDADKGPWCFTT
DPSVRWEYCNLKKCSGTEASVVAPPPVVLLPDVETPSEEDCMFGNGKGYRGKRATTVTGTPCQDWAAQ
EPHRHSIFTPETNPRAGLEKNYCRNPDGDVGGPWCYTTNPRKLYDYCDVPQCAAPSFDCGKPQVEPKKC
PGRVVGGCVAHPHSWPWQVSLRTRFGMHFCGGTLISPEWVLTAAHCLEKSPRPSSYKVILGAHQEVNLEP
HVQEIEVSRLFLEPTRKDIALLKLSSPAVITDKVIPACLPSPNYVVADRTECFITGWGETQGTFGAGLLKEA
QLPVIENKVCNRYEFLNGRVQSTELCAGHLAGGTDSCQGDSGGPLVCFEKDKYILQGVTSWGLGCARPN
KPGVYVRVSRFVTWIEGVMRNN
SEQ ID NO: 7 (a nucleic sequence of delta-plg (delta-plasminogen)): 
gagcctctggatgactatgtgaatacccagggggcttcactgttcagtgtcactaagaagcagctgggagcaggaagtatagaagaatgtgcagcaaaatgtgagga
ggacgaagaattcacctgcagggcattccaatatcacagtaaagagcaacaatgtgtgataatggctgaaaacaggaagtcctccataatcattaggatgagagatgt
agttttatttgaaaagaaagtgtatctctcagagtgcaagactgggaatggaaagaactacagagggacgatgtccaaaacaaaaaatggcatcacctgtcaaaaatg
gagttccacttctccccacagacctagattctcacctgctacacacccctcagagggactggaggagaactactgcaggaatccagacaacgatccgcaggggccct
ggtgctatactactgatccagaaaagagatatgactactgcgacattcttgagtgtgaagaggcggcccccttcatttgattgtgggaagcctcaagtggagccgaagaa
atgtcctggaagggttgtagggggggtgtgtggcccacccacattcctggccctggcaagtcagtcttagaacaaggtttggaatgcacttctgtggaggcaccttgata
tccccagagtgggtgttgactgctgcccactgcttggagaagtccccaaggccttcatcctacaaggtcatcctgggtgcacaccaagaagtgaatctcgaaccgcat
gttcaggaaatagaagtgtctaggctgttcttggagcccacacgaaaagatattgccttgctaaagctaagcagtcctgccgtcatcactgacaaagtaatcccagcttg
tctgccatccccaaattatgtggtcgctgaccggaccgaatgtttcatcactggctggggagaaacccaaggtacttttggagctggccttctcaaggaagcccagctc
cctgtgattgagaataaagtgtgcaatcgctatgagtttctgaatggaagagtccaatccaccgaactctgtgctgggcatttggccggaggcactgacagttgccagg
 
gtgacagtggaggtcctctggtttgcttcgagaaggacaaatacattttacaaggagtcacttcttggggtcttggctgtgcacgccccaataagcctggtgtctatgttc
gtgtttcaaggtttgttacttggattgagggagtgatgagaaataattaa
SEQ ID NO: 8 (an amino acid sequence of delta-plg (delta-plasminogen)): 
EPLDDYVNTQGASLFSVTKKQLGAGSIEECAAKCEEDEEFTCRAFQYHSKEQQCVIMAENRKSSIIIRMR
DVVLFEKKVYLSECKTGNGKNYRGTMSKTKNGITCQKWSSTSPHRPRFSPATHPSEGLEENYCRNPDND
PQGPWCYTTDPEKRYDYCDILECEEAAPSFDCGKPQVEPKKCPGRVVGGCVAHPHSWPWQVSLRTRFG
MHFCGGTLISPEWVLTAAHCLEKSPRPSSYKVILGAHQEVNLEPHVQEIEVSRLFLEPTRKDIALLKLSSPA
VITDKVIPACLPSPNYVVADRTECFITGWGETQGTFGAGLLKEAQLPVIENKVCNRYEFLNGRVQSTELCA
GHLAGGTDSCQGDSGGPLVCFEKDKYILQGVTSWGLGCARPNKPGVYVRVSRFVTWIEGVMRNN
SEQ ID NO: 9(a nucleic sequence of mini-plg (mini-plasminogen)): 
gtcaggtgggagtactgcaacctgaaaaaatgctcaggaacagaagcgagtgttgtagcacctccgcctgttgtcctgcttccagatgtagagactccttccgaagaa
gactgtatgtttgggaatgggaaaggataccgaggcaagagggcgaccactgttactgggacgccatgccaggactgggctgcccaggagccccatagacacag
cattttcactccagagacaaatccacgggcgggtctggaaaaaaattactgccgtaaccctgatggtgatgtaggtggtccctggtgctacacgacaaatccaagaaa
actttacgactactgtgatgtccctcagtgtgcggccccttcatttgattgtgggaagcctcaagtggagccgaagaaatgtcctggaagggttgtaggggggtgtgtg
gcccacccacattcctggccctggcaagtcagtcttagaacaaggtttggaatgcacttctgtggaggcaccttgatatccccagagtgggtgttgactgctgcccact
gcttggagaagtccccaaggccttcatcctacaaggtcatcctgggtgcacaccaagaagtgaatctcgaaccgcatgttcaggaaatagaagtgtctaggctgttctt
ggagcccacacgaaaagatattgccttgctaaagctaagcagtcctgccgtcatcactgacaaagtaatcccagcttgtctgccatccccaaattatgtggtcgctgac
cggaccgaatgtttcatcactggctggggagaaacccaaggtacttttggagctggccttctcaaggaagcccagctccctgtgattgagaataaagtgtgcaatcgct
atgagtttctgaatggaagagtccaatccaccgaactctgtgctgggcatttggccggaggcactgacagttgccagggtgacagtggaggtcctctggtttgcttcga
gaaggacaaatacattttacaaggagtcacttcttggggtcttggctgtgcacgccccaataagcctggtgtctatgttcgtgtttcaaggtttgttacttggattgaggga
gtgatgagaaataattaa
SEQ ID NO: 10 (an amino acid sequence of mini-plg (mini-plasminogen)): 
VRWEYCNLKKCSGTEASVVAPPPVVLLPDVETPSEEDCMFGNGKGYRGKRATTVTGTPCQDWAAQEPH
RHSIFTPETNPRAGLEKNYCRNPDGDVGGPWCYTTNPRKLYDYCDVPQCAAPSFDCGKPQVEPKKCPGR
VVGGCVAHPHSWPWQVSLRTRFGMHFCGGTLISPEWVLTAAHCLEKSPRPSSYKVILGAHQEVNLEPHV
QEIEVSRLFLEPTRKDIALLKLSSPAVITDKVIPACLPSPNYVVADRTECFITGWGETQGTFGAGLLKEAQLP
VIENKVCNRYEFLNGRVQSTELCAGHLAGGTDSCQGDSGGPLVCFEKDKYILQGVTSWGLGCARPNKPG
VYVRVSRFVTWIEGVMRNN
SEQ ID NO: 11 (a nucleic sequence of micro-plg (micro-plasminogen)): 
gccccttcatttgattgtgggaagcctcaagtggagccgaagaaatgtcctggaagggttgtaggggggtgtgtggcccacccacattcctggccctggcaagtcagt
cttagaacaaggtttggaatgcacttctgtggaggcaccttgatatccccagagtgggtgttgactgctgcccactgcttggagaagtccccaaggccttcatcctacaa
ggtcatcctgggtgcacaccaagaagtgaatctcgaaccgcatgttcaggaaatagaagtgtctaggctgttcttggagcccacacgaaaagatattgccttgctaaag
ctaagcagtcctgccgtcatcactgacaaagtaatcccagcttgtctgccatccccaaattatgtggtcgctgaccggaccgaatgtttcatcactggctggggagaaa
cccaaggtacttttggagctggccttctcaaggaagcccagctccctgtgattgagaataaagtgtgcaatcgctatgagtttctgaatggaagagtccaatccaccgaa
ctctgtgctgggcatttggccggaggcactgacagttgccagggtgacagtggaggtcctctggtttgcttcgagaaggacaaatacattttacaaggagtcacttcttg
gggtcttggctgtgcacgccccaataagcctggtgtctatgttcgtgtttcaaggtttgttacttggattgagggagtgatgagaaataattaa
SEQ ID NO: 12 (an amino acid sequence of micro-plg (micro-plasminogen)): 
APSFDCGKPQVEPKKCPGRVVGGCVAHPHSWPWQVSLRTRFGMHFCGGTLISPEWVLTAAHCLEKSPRP
SSYKVILGAHQEVNLEPHVQEIEVSRLFLEPTRKDIALLKLSSPAVITDKVIPACLPSPNYVVADRTECFITG
WGETQGTFGAGLLKEAQLPVIENKVCNRYEFLNGRVQSTELCAGHLAGGTDSCQGDSGGPLVCFEKDKY
 ILQGVTSWGLGCARPNKPGVYVRVSRFVTWIEGVMRNN
SEQ ID NO: 13 (a nucleic sequence of serine protease domain): 
gttgtaggggggtgtgtggcccacccacattcctggccctggcaagtcagtcttagaacaaggtttggaatgcacttctgtggaggcaccttgatatccccagagtggg
tgttgactgctgcccactgcttggagaagtccccaaggccttcatcctacaaggtcatcctgggtgcacaccaagaagtgaatctcgaaccgcatgttcaggaaataga
agtgtctaggctgttcttggagcccacacgaaaagatattgccttgctaaagctaagcagtcctgccgtcatcactgacaaagtaatcccagcttgtctgccatccccaa
attatgtggtcgctgaccggaccgaatgtttc360
atcactggctggggagaaacccaaggtacttttggagctggccttctcaaggaagcccagctccctgtgattgagaataaagtgtgcaatcgctatgagtttctgaatg
gaagagtccaatccaccgaactctgtgctgggcatttggccggaggcactgacagttgccagggtgacagtggaggtcctctggtttgcttcgagaaggacaaatac
attttacaaggagtcacttcttggggtcttggctgtgcacgccccaataagcctggtgtctatgttcgtgtttcaaggtttgttacttggattgagggagtgatgaga
SEQ ID NO: 14 (an amino acid sequence of serine protease domain):

VVGGCVAHPHSWPWQVSLRTRFGMHFCGGTLISPEWVLTAAHCLEKSPRPSSYKVILGAHQEVNLEPHV
QEIEVSRLFLEPTRKDIALLKLSSPAVITDKVIPACLPSPNYVVADRTECFITGWGETQGTFGAGLLKEAQLP
VIENKVCNRYEFLNGRVQSTELCAGHLAGGTDSCQGDSGGPLVCFEKDKYILQGVTSWGLGCARPNKPG
VYVRVSRFVTWIEGVMR

## Claims

1. A method for promoting degradation of pathological TDP-43 protein, comprising administering to a subject a therapeutically effective amount of one or more compounds selected from the group consisting of: a component of the plasminogen activation pathway, a compound capable of directly activating plasminogen or indirectly activating plasminogen by activating a upstream component of the plasminogen activation pathway, a compound simulating the activity of plasminogen or plasmin, a compound capable of upregulating the expression of plasminogen or plasminogen activator, a plasminogen analog, a plasmin analog, a tPA or uPA analog, and an antagonist of fibrinolysis inhibitor.

2. The method according to claim 1, wherein the component of the plasminogen activation pathway is selected from the group consisting of: plasminogen, recombinant human plasmin, Lys-plasminogen, Glu-plasminogen, plasmin, plasminogen and plasmin variants and analogs comprising one or more kringle domains and a protease domain of plasminogen and plasmin, mini-plasminogen, mini-plasmin, micro-plasminogen, micro-plasmin, delta-plasminogen, delta-plasmin, plasminogen activator, tPA and uPA.

3. The method according to claim 1, wherein the antagonist of the fibrinolysis inhibitor is an inhibitor of PAI-1, complement C1 inhibitor, α2 antiplasmin or α2 macroglobulin, such as an antibody.

4. The method according to any one of claims 1-3, wherein the compound has one or more activities selected from the group consisting of: promoting the degradation of pathological TDP-43 protein in nerve tissue, promoting the degradation of pathological TDP-43 protein in muscle tissue, and promoting the degradation of TDP-43 in cells and cell nuclei.

5. A method for treating a pathological TDP-43 protein-related disease in a subject, comprising administering to the subject a therapeutically effective amount of one or more compounds selected from the group consisting of: a component of the plasminogen activation pathway, a compound capable of directly activating plasminogen or indirectly activating plasminogen by activating a upstream component of the plasminogen activation pathway, a compound simulating the activity of plasminogen or plasmin, a compound capable of upregulating the expression of plasminogen or plasminogen activator, a plasminogen analog, a plasmin analog, a tPA or uPA analog, and an antagonist of fibrinolysis inhibitor, wherein the pathological TDP-43 protein-related disease is one or more selected from the group consisting of: amyotrophic lateral sclerosis (ALS), bulbar amyotrophic lateral sclerosis, Fus gene mutation amyotrophic lateral sclerosis, Alzheimer's disease, argyrophilic grain disease, ALS-parkinsonism dementia complex of Guam, vascular dementia, frontotemporal dementia (FTD), semantic dementia, dementia with Lewy bodies, Huntington's disease, spinocere bellarataxia, inclusion body myopathy, inclusion body myositis, and Parkinson's disease.

6. The method according to claim 5, wherein the component of the plasminogen activation pathway is selected from the group consisting of: plasminogen, recombinant human plasmin, Lys-plasminogen, Glu-plasminogen, plasmin, plasminogen and plasmin variants and analogs comprising one or more kringle domains and a protease domain of plasminogen and plasmin, mini-plasminogen, mini-plasmin, micro-plasminogen, micro-plasmin, delta-plasminogen, delta-plasmin, plasminogen activator, tPA and uPA.

7. The method according to claim 5, wherein the antagonist of the fibrinolysis inhibitor is an inhibitor of PAI-1, complement C1 inhibitor, α2 antiplasmin or α2 macroglobulin, such as an antibody.

8. The method according to any one of claims 1-7, wherein the compound is plasminogen or plasmin.

9. The method according to any one of claims 1-8, wherein the plasminogen is Glu-plasminogen, Lys-plasminogen, or a conservatively substituted variant thereof.

10. The method according to any one of claims 1-9, wherein the plasminogen has at least 75%, 80%, 85%, 90%, 95%, 96%, 97%, 98% or 99% sequence identity with SEQ ID NO: 2 and has lysine binding activity and/or proteolytic activity of plasminogen.

11. The method according to any one of claims 1-10, wherein the plasminogen comprises one or more domains selected from the group consisting of:
1) a serine protease domain represented by SEQ ID NO:14;
2) a serine protease domain having at least 80%, 90%, 95%, 96%, 97%, 98%, 99% identity with SEQ ID NO: 14 and retaining proteolytic activity;
3) one or more Kringle domains selected from the group consisting of: Kringle 1, Kringle 2, Kringle 3, Kringle 4 and Kringle 5; and
4) a Kringle domain having at least 80%, 90%, 95%, 96%, 97%, 98%, 99% identity with one or more of Kringle 1, Kringle 2, Kringle 3, Kringle 4, and Kringle 5 and retaining lysine binding activity.

12. The method according to any one of claims 1-11, wherein the plasminogen is selected from Glu-plasminogen, Lys-plasminogen, mini-plasminogen, micro-plasminogen, delta-plasminogen, or variants thereof retaining the proteolytic activity of plasminogen.

13. The method according to any one of claims 1-12, wherein the plasminogen comprises an amino acid sequence represented by SEQ ID NO: 2, 6, 8, 10 or 12, or a conservatively substituted variant of the amino acid sequence represented by SEQ ID NO: 2, 6, 8, 10 or 12.

14. The method according to any one of claims 1-13, wherein the plasminogen is used in combination with one or more other therapies or drugs.

15. The method according to claim 14, wherein the other therapies include cell therapy (including stem cell therapy), supportive therapy and physical therapy.

16. The method according to any one of claims 1-15, wherein the plasminogen is administered by nasal inhalation, nebulized inhalation, nasal drops, eye drops, ear drops, or intravenous, intraperitoneal, subcutaneous, sublingual, intracranial, intrathecal, intraarterial or intramuscular administration.
